# EUROPEAN PATENT APPLICATION

(11) **EP 3 912 681 A1**
(43) Date of publication of application: **24.11.2021**
(21) Application number: 21165971.9
(22) Date of filing: 14.03.2017
(51) Int. Cl.: A61P 35/00, C07K 16/28, C07K 16/30, C07K 16/40

(54) **ANTI-CD39 ANTIBODIES**

(30) Priority: 14.03.2016 US 201662307606 P; 06.10.2016 US 201662404780 P
(62) Divisional of application: 17710894.1
(71) Applicant: Orega Biotech, 69130 Ecully (FR)
(72) Inventor: GAUTHIER, Laurent, 13008 MARSEILLE (FR); PATUREL, Carine, 69280 MARCY L'ETOILE (FR); PERROT, Ivan, 13260 CASSIS (FR)
(74) Representative: Cabinet Laurent & Charras

(57) **Abstract**

The present invention relates to antigen-binding compounds that inhibit CD39. The invention also relates to cells producing such compounds; methods of making such compounds, and antibodies, fragments, variants, and derivatives thereof; pharmaceutical compositions comprising the same; methods of using the compounds to diagnose, treat or prevent diseases, e.g. cancer.

SEQUENCE LISTING

<110>  OREGA BIOTECH

<120>  ANTI-CD39 ANTIBODIES

<130>  057-B-59304 EP DIV

<140>  PCT/EP2017/056011
<141>  2017-03-14

<150>  US 62/307,606
<151>  2016-03-14

<150>  US 62/404,780
<151>  2016-10-06

<160>  35

<170>  PatentIn version 3.5

<210>  1
<211>  510
<212>  PRT
<213>  homo sapiens

<400>  1

Met Glu Asp Thr Lys Glu Ser Asn Val Lys Thr Phe Cys Ser Lys Asn
1               5                   10                  15

Ile Leu Ala Ile Leu Gly Phe Ser Ser Ile Ile Ala Val Ile Ala Leu
            20                  25                  30

Leu Ala Val Gly Leu Thr Gln Asn Lys Ala Leu Pro Glu Asn Val Lys
        35                  40                  45

Tyr Gly Ile Val Leu Asp Ala Gly Ser Ser His Thr Ser Leu Tyr Ile
    50                  55                  60

Tyr Lys Trp Pro Ala Glu Lys Glu Asn Asp Thr Gly Val Val His Gln
65                  70                  75                  80

Val Glu Glu Cys Arg Val Lys Gly Pro Gly Ile Ser Lys Phe Val Gln
                85                  90                  95

Lys Val Asn Glu Ile Gly Ile Tyr Leu Thr Asp Cys Met Glu Arg Ala
            100                 105                 110

Arg Glu Val Ile Pro Arg Ser Gln His Gln Glu Thr Pro Val Tyr Leu
        115                 120                 125

Gly Ala Thr Ala Gly Met Arg Leu Leu Arg Met Glu Ser Glu Glu Leu
    130                 135                 140

Ala Asp Arg Val Leu Asp Val Val Glu Arg Ser Leu Ser Asn Tyr Pro
145                 150                 155                 160

Phe Asp Phe Gln Gly Ala Arg Ile Ile Thr Gly Gln Glu Glu Gly Ala
                165                 170                 175

Tyr Gly Trp Ile Thr Ile Asn Tyr Leu Leu Gly Lys Phe Ser Gln Lys
            180                 185                 190

Thr Arg Trp Phe Ser Ile Val Pro Tyr Glu Thr Asn Asn Gln Glu Thr
        195                 200                 205

Phe Gly Ala Leu Asp Leu Gly Gly Ala Ser Thr Gln Val Thr Phe Val
    210                 215                 220

Pro Gln Asn Gln Thr Ile Glu Ser Pro Asp Asn Ala Leu Gln Phe Arg
225                 230                 235                 240

Leu Tyr Gly Lys Asp Tyr Asn Val Tyr Thr His Ser Phe Leu Cys Tyr
                245                 250                 255

Gly Lys Asp Gln Ala Leu Trp Gln Lys Leu Ala Lys Asp Ile Gln Val
            260                 265                 270

Ala Ser Asn Glu Ile Leu Arg Asp Pro Cys Phe His Pro Gly Tyr Lys
        275                 280                 285

Lys Val Val Asn Val Ser Asp Leu Tyr Lys Thr Pro Cys Thr Lys Arg
    290                 295                 300

Phe Glu Met Thr Leu Pro Phe Gln Gln Phe Glu Ile Gln Gly Ile Gly
305                 310                 315                 320

Asn Tyr Gln Gln Cys His Gln Ser Ile Leu Glu Leu Phe Asn Thr Ser
                325                 330                 335

Tyr Cys Pro Tyr Ser Gln Cys Ala Phe Asn Gly Ile Phe Leu Pro Pro
            340                 345                 350

Leu Gln Gly Asp Phe Gly Ala Phe Ser Ala Phe Tyr Phe Val Met Lys
        355                 360                 365

Phe Leu Asn Leu Thr Ser Glu Lys Val Ser Gln Glu Lys Val Thr Glu
    370                 375                 380

Met Met Lys Lys Phe Cys Ala Gln Pro Trp Glu Glu Ile Lys Thr Ser
385                 390                 395                 400

Tyr Ala Gly Val Lys Glu Lys Tyr Leu Ser Glu Tyr Cys Phe Ser Gly
                405                 410                 415

Thr Tyr Ile Leu Ser Leu Leu Leu Gln Gly Tyr His Phe Thr Ala Asp
            420                 425                 430

Ser Trp Glu His Ile His Phe Ile Gly Lys Ile Gln Gly Ser Asp Ala
        435                 440                 445

Gly Trp Thr Leu Gly Tyr Met Leu Asn Leu Thr Asn Met Ile Pro Ala
    450                 455                 460

Glu Gln Pro Leu Ser Thr Pro Leu Ser His Ser Thr Tyr Val Phe Leu
465                 470                 475                 480

Met Val Leu Phe Ser Leu Val Leu Phe Thr Val Ala Ile Ile Gly Leu
                485                 490                 495

Leu Ile Phe His Lys Pro Ser Tyr Phe Trp Lys Asp Met Val
            500                 505                 510

<210>  2
<211>  472
<212>  PRT
<213>  homo sapiens

<400>  2

Met Ala Gly Lys Val Arg Ser Leu Leu Pro Pro Leu Leu Leu Ala Ala
1               5                   10                  15

Ala Gly Leu Ala Gly Leu Leu Leu Leu Cys Val Pro Thr Arg Asp Val
            20                  25                  30

Arg Glu Pro Pro Ala Leu Lys Tyr Gly Ile Val Leu Asp Ala Gly Ser
        35                  40                  45

Ser His Thr Ser Met Phe Ile Tyr Lys Trp Pro Ala Asp Lys Glu Asn
    50                  55                  60

Asp Thr Gly Ile Val Gly Gln His Ser Ser Cys Asp Val Pro Gly Gly
65                  70                  75                  80

Gly Ile Ser Ser Tyr Ala Asp Asn Pro Ser Gly Ala Ser Gln Ser Leu
                85                  90                  95

Val Gly Cys Leu Glu Gln Ala Leu Gln Asp Val Pro Lys Glu Arg His
            100                 105                 110

Ala Gly Thr Pro Leu Tyr Leu Gly Ala Thr Ala Gly Met Arg Leu Leu
        115                 120                 125

Asn Leu Thr Asn Pro Glu Ala Ser Thr Ser Val Leu Met Ala Val Thr
    130                 135                 140

His Thr Leu Thr Gln Tyr Pro Phe Asp Phe Arg Gly Ala Arg Ile Leu
145                 150                 155                 160

Ser Gly Gln Glu Glu Gly Val Phe Gly Trp Val Thr Ala Asn Tyr Leu
                165                 170                 175

Leu Glu Asn Phe Ile Lys Tyr Gly Trp Val Gly Arg Trp Phe Arg Pro
            180                 185                 190

Arg Lys Gly Thr Leu Gly Ala Met Asp Leu Gly Gly Ala Ser Thr Gln
        195                 200                 205

Ile Thr Phe Glu Thr Thr Ser Pro Ala Glu Asp Arg Ala Ser Glu Val
    210                 215                 220

Gln Leu His Leu Tyr Gly Gln His Tyr Arg Val Tyr Thr His Ser Phe
225                 230                 235                 240

Leu Cys Tyr Gly Arg Asp Gln Val Leu Gln Arg Leu Leu Ala Ser Ala
                245                 250                 255

Leu Gln Thr His Gly Phe His Pro Cys Trp Pro Arg Gly Phe Ser Thr
            260                 265                 270

Gln Val Leu Leu Gly Asp Val Tyr Gln Ser Pro Cys Thr Met Ala Gln
        275                 280                 285

Arg Pro Gln Asn Phe Asn Ser Ser Ala Arg Val Ser Leu Ser Gly Ser
    290                 295                 300

Ser Asp Pro His Leu Cys Arg Asp Leu Val Ser Gly Leu Phe Ser Phe
305                 310                 315                 320

Ser Ser Cys Pro Phe Ser Arg Cys Ser Phe Asn Gly Val Phe Gln Pro
                325                 330                 335

Pro Val Ala Gly Asn Phe Val Ala Phe Ser Ala Phe Phe Tyr Thr Val
            340                 345                 350

Asp Phe Leu Arg Thr Ser Met Gly Leu Pro Val Ala Thr Leu Gln Gln

355                 360                 365

Leu Glu Ala Ala Ala Val Asn Val Cys Asn Gln Thr Trp Ala Gln Gln
    370                 375                 380

Leu Leu Ser Arg Gly Tyr Gly Phe Asp Glu Arg Ala Phe Gly Gly Val
385                 390                 395                 400

Ile Phe Gln Lys Lys Ala Ala Asp Thr Ala Val Gly Trp Ala Leu Gly
                405                 410                 415

Tyr Met Leu Asn Leu Thr Asn Leu Ile Pro Ala Asp Pro Pro Gly Leu
            420                 425                 430

Arg Lys Gly Thr Asp Phe Ser Ser Trp Val Val Leu Leu Leu Leu Phe
        435                 440                 445

Ala Ser Ala Leu Leu Ala Ala Leu Val Leu Leu Leu Arg Gln Val His
    450                 455                 460

Ser Ala Lys Leu Pro Ser Thr Ile
465                 470

<210>  3
<211>  484
<212>  PRT
<213>  homo sapiens

<400>  3

Met Lys Lys Gly Ile Arg Tyr Glu Thr Ser Arg Lys Thr Ser Tyr Ile
1               5                   10                  15

Phe Gln Gln Pro Gln His Gly Pro Trp Gln Thr Arg Met Arg Lys Ile
            20                  25                  30

Ser Asn His Gly Ser Leu Arg Val Ala Lys Val Ala Tyr Pro Leu Gly
        35                  40                  45

Leu Cys Val Gly Val Phe Ile Tyr Val Ala Tyr Ile Lys Trp His Arg
    50                  55                  60

Ala Thr Ala Thr Gln Ala Phe Phe Ser Ile Thr Arg Ala Ala Pro Gly
65                  70                  75                  80

Ala Arg Trp Gly Gln Gln Ala His Ser Pro Leu Gly Thr Ala Ala Asp
                85                  90                  95

Gly His Glu Val Phe Tyr Gly Ile Met Phe Asp Ala Gly Ser Thr Gly

100                 105                 110

Thr Arg Val His Val Phe Gln Phe Thr Arg Pro Pro Arg Glu Thr Pro
        115                 120                 125

Thr Leu Thr His Glu Thr Phe Lys Ala Leu Lys Pro Gly Leu Ser Ala
    130                 135                 140

Tyr Ala Asp Asp Val Glu Lys Ser Ala Gln Gly Ile Arg Glu Leu Leu
145                 150                 155                 160

Asp Val Ala Lys Gln Asp Ile Pro Phe Asp Phe Trp Lys Ala Thr Pro
                165                 170                 175

Leu Val Leu Lys Ala Thr Ala Gly Leu Arg Leu Leu Pro Gly Glu Lys
            180                 185                 190

Ala Gln Lys Leu Leu Gln Lys Val Lys Glu Val Phe Lys Ala Ser Pro
        195                 200                 205

Phe Leu Val Gly Asp Asp Cys Val Ser Ile Met Asn Gly Thr Asp Glu
    210                 215                 220

Gly Val Ser Ala Trp Ile Thr Ile Asn Phe Leu Thr Gly Ser Leu Lys
225                 230                 235                 240

Thr Pro Gly Gly Ser Ser Val Gly Met Leu Asp Leu Gly Gly Gly Ser
                245                 250                 255

Thr Gln Ile Ala Phe Leu Pro Arg Val Glu Gly Thr Leu Gln Ala Ser
            260                 265                 270

Pro Pro Gly Tyr Leu Thr Ala Leu Arg Met Phe Asn Arg Thr Tyr Lys
        275                 280                 285

Leu Tyr Ser Tyr Ser Tyr Leu Gly Leu Gly Leu Met Ser Ala Arg Leu
    290                 295                 300

Ala Ile Leu Gly Gly Val Glu Gly Gln Pro Ala Lys Asp Gly Lys Glu
305                 310                 315                 320

Leu Val Ser Pro Cys Leu Ser Pro Ser Phe Lys Gly Glu Trp Glu His
                325                 330                 335

Ala Glu Val Thr Tyr Arg Val Ser Gly Gln Lys Ala Ala Ala Ser Leu
            340                 345                 350

His Glu Leu Cys Ala Ala Arg Val Ser Glu Val Leu Gln Asn Arg Val
        355                 360                 365

His Arg Thr Glu Glu Val Lys His Val Asp Phe Tyr Ala Phe Ser Tyr
    370                 375                 380

Tyr Tyr Asp Leu Ala Ala Gly Val Gly Leu Ile Asp Ala Glu Lys Gly
385                 390                 395                 400

Gly Ser Leu Val Val Gly Asp Phe Glu Ile Ala Ala Lys Tyr Val Cys
                405                 410                 415

Arg Thr Leu Glu Thr Gln Pro Gln Ser Ser Pro Phe Ser Cys Met Asp
            420                 425                 430

Leu Thr Tyr Val Ser Leu Leu Leu Gln Glu Phe Gly Phe Pro Arg Ser
        435                 440                 445

Lys Val Leu Lys Leu Thr Arg Lys Ile Asp Asn Val Glu Thr Ser Trp
    450                 455                 460

Ala Leu Gly Ala Ile Phe His Tyr Ile Asp Ser Leu Asn Arg Gln Lys
465                 470                 475                 480

Ser Pro Ala Ser

<210>  4
<211>  529
<212>  PRT
<213>  homo sapiens

<400>  4

Met Phe Thr Val Leu Thr Arg Gln Pro Cys Glu Gln Ala Gly Leu Lys
1               5                   10                  15

Ala Leu Tyr Arg Thr Pro Thr Ile Ile Ala Leu Val Val Leu Leu Val
            20                  25                  30

Ser Ile Val Val Leu Val Ser Ile Thr Val Ile Gln Ile His Lys Gln
        35                  40                  45

Glu Val Leu Pro Pro Gly Leu Lys Tyr Gly Ile Val Leu Asp Ala Gly
    50                  55                  60

Ser Ser Arg Thr Thr Val Tyr Val Tyr Gln Trp Pro Ala Glu Lys Glu
65                  70                  75                  80

Asn Asn Thr Gly Val Val Ser Gln Thr Phe Lys Cys Ser Val Lys Gly
                85                  90                  95

Ser Gly Ile Ser Ser Tyr Gly Asn Asn Pro Gln Asp Val Pro Arg Ala
            100                 105                 110

Phe Glu Glu Cys Met Gln Lys Val Lys Gly Gln Val Pro Ser His Leu
        115                 120                 125

His Gly Ser Thr Pro Ile His Leu Gly Ala Thr Ala Gly Met Arg Leu
    130                 135                 140

Leu Arg Leu Gln Asn Glu Thr Ala Ala Asn Glu Val Leu Glu Ser Ile
145                 150                 155                 160

Gln Ser Tyr Phe Lys Ser Gln Pro Phe Asp Phe Arg Gly Ala Gln Ile
                165                 170                 175

Ile Ser Gly Gln Glu Glu Gly Val Tyr Gly Trp Ile Thr Ala Asn Tyr
            180                 185                 190

Leu Met Gly Asn Phe Leu Glu Lys Asn Leu Trp His Met Trp Val His
        195                 200                 205

Pro His Gly Val Glu Thr Thr Gly Ala Leu Asp Leu Gly Gly Ala Ser
    210                 215                 220

Thr Gln Ile Ser Phe Val Ala Gly Glu Lys Met Asp Leu Asn Thr Ser
225                 230                 235                 240

Asp Ile Met Gln Val Ser Leu Tyr Gly Tyr Val Tyr Thr Leu Tyr Thr
                245                 250                 255

His Ser Phe Gln Cys Tyr Gly Arg Asn Glu Ala Glu Lys Lys Phe Leu
            260                 265                 270

Ala Met Leu Leu Gln Asn Ser Pro Thr Lys Asn His Leu Thr Asn Pro
        275                 280                 285

Cys Tyr Pro Arg Asp Tyr Ser Ile Ser Phe Thr Met Gly His Val Phe
    290                 295                 300

Asp Ser Leu Cys Thr Val Asp Gln Arg Pro Glu Ser Tyr Asn Pro Asn
305                 310                 315                 320

Asp Val Ile Thr Phe Glu Gly Thr Gly Asp Pro Ser Leu Cys Lys Glu
                325                 330                 335

Lys Val Ala Ser Ile Phe Asp Phe Lys Ala Cys His Asp Gln Glu Thr
            340                 345                 350

Cys Ser Phe Asp Gly Val Tyr Gln Pro Lys Ile Lys Gly Pro Phe Val
        355                 360                 365

Ala Phe Ala Gly Phe Tyr Tyr Thr Ala Ser Ala Leu Asn Leu Ser Gly
    370                 375                 380

Ser Phe Ser Leu Asp Thr Phe Asn Ser Ser Thr Trp Asn Phe Cys Ser
385                 390                 395                 400

Gln Asn Trp Ser Gln Leu Pro Leu Leu Leu Pro Lys Phe Asp Glu Val
                405                 410                 415

Tyr Ala Arg Ser Tyr Cys Phe Ser Ala Asn Tyr Ile Tyr His Leu Phe
            420                 425                 430

Val Asn Gly Tyr Lys Phe Thr Glu Glu Thr Trp Pro Gln Ile His Phe
        435                 440                 445

Glu Lys Glu Val Gly Asn Ser Ser Ile Ala Trp Ser Leu Gly Tyr Met
    450                 455                 460

Leu Ser Leu Thr Asn Gln Ile Pro Ala Glu Ser Pro Leu Ile Arg Leu
465                 470                 475                 480

Pro Ile Glu Pro Pro Val Phe Val Gly Thr Leu Ala Phe Phe Thr Ala
                485                 490                 495

Ala Ala Leu Leu Cys Leu Ala Phe Leu Ala Tyr Leu Cys Ser Ala Thr
            500                 505                 510

Arg Arg Lys Arg His Ser Glu His Ala Phe Asp His Ala Val Asp Ser
        515                 520                 525

Asp

<210> 5
<211> 428
<212> PRT
<213> homo sapiens

<400> 5

Met Ala Thr Ser Trp Gly Thr Val Phe Phe Met Leu Val Val Ser Cys
1               5                   10                  15

Val Cys Ser Ala Val Ser His Arg Asn Gln Gln Thr Trp Phe Glu Gly
            20                  25                  30

Ile Phe Leu Ser Ser Met Cys Pro Ile Asn Val Ser Ala Ser Thr Leu
        35                  40                  45

Tyr Gly Ile Met Phe Asp Ala Gly Ser Thr Gly Thr Arg Ile His Val
    50                  55                  60

Tyr Thr Phe Val Gln Lys Met Pro Gly Gln Leu Pro Ile Leu Glu Gly
65                  70                  75                  80

Glu Val Phe Asp Ser Val Lys Pro Gly Leu Ser Ala Phe Val Asp Gln
                85                  90                  95

Pro Lys Gln Gly Ala Glu Thr Val Gln Gly Leu Leu Glu Val Ala Lys
            100                 105                 110

Asp Ser Ile Pro Arg Ser His Trp Lys Lys Thr Pro Val Val Leu Lys
        115                 120                 125

Ala Thr Ala Gly Leu Arg Leu Leu Pro Glu His Lys Ala Lys Ala Leu
    130                 135                 140

Leu Phe Glu Val Lys Glu Ile Phe Arg Lys Ser Pro Phe Leu Val Pro
145                 150                 155                 160

Lys Gly Ser Val Ser Ile Met Asp Gly Ser Asp Glu Gly Ile Leu Ala
                165                 170                 175

Trp Val Thr Val Asn Phe Leu Thr Gly Gln Leu His Gly His Arg Gln
            180                 185                 190

Glu Thr Val Gly Thr Leu Asp Leu Gly Gly Ala Ser Thr Gln Ile Thr
        195                 200                 205

Phe Leu Pro Gln Phe Glu Lys Thr Leu Glu Gln Thr Pro Arg Gly Tyr
    210                 215                 220

Leu Thr Ser Phe Glu Met Phe Asn Ser Thr Tyr Lys Leu Tyr Thr His
225                 230                 235                 240

Ser Tyr Leu Gly Phe Gly Leu Lys Ala Ala Arg Leu Ala Thr Leu Gly
                245                 250                 255

Ala Leu Glu Thr Glu Gly Thr Asp Gly His Thr Phe Arg Ser Ala Cys
            260                 265                 270

Leu Pro Arg Trp Leu Glu Ala Glu Trp Ile Phe Gly Gly Val Lys Tyr
        275                 280                 285

Gln Tyr Gly Gly Asn Gln Glu Gly Glu Val Gly Phe Glu Pro Cys Tyr
    290                 295                 300

Ala Glu Val Leu Arg Val Val Arg Gly Lys Leu His Gln Pro Glu Glu
305                 310                 315                 320

Val Gln Arg Gly Ser Phe Tyr Ala Phe Ser Tyr Tyr Tyr Asp Arg Ala
                325                 330                 335

Val Asp Thr Asp Met Ile Asp Tyr Glu Lys Gly Gly Ile Leu Lys Val
            340                 345                 350

Glu Asp Phe Glu Arg Lys Ala Arg Glu Val Cys Asp Asn Leu Glu Asn
        355                 360                 365

Phe Thr Ser Gly Ser Pro Phe Leu Cys Met Asp Leu Ser Tyr Ile Thr
    370                 375                 380

Ala Leu Leu Lys Asp Gly Phe Gly Phe Ala Asp Ser Thr Val Leu Gln
385                 390                 395                 400

Leu Thr Lys Lys Val Asn Asn Ile Glu Thr Gly Trp Ala Leu Gly Ala
                405                 410                 415

Thr Phe His Leu Leu Gln Ser Leu Gly Ile Ser His
            420                 425

<210>  6
<211>  123
<212>  PRT
<213>  Artificial

<220>
<223>  Chimeric

<400>  6

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr His Tyr
            20                  25                  30

Gly Met Asn Trp Val Arg Gln Ala Pro Gly Gln Gly Leu Glu Trp Met
        35                  40                  45

Gly Trp Ile Asn Thr Tyr Thr Gly Glu Pro Thr Tyr Ala Asp Asp Phe
    50                  55                  60

Lys Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Arg Arg Arg Tyr Glu Gly Asn Tyr Val Phe Tyr Tyr Phe Asp Tyr
            100                 105                 110

Trp Gly Gln Gly Thr Thr Val Thr Val Ser Ser
        115                 120

<210>  7
<211>  107
<212>  PRT
<213>  Artificial

<220>
<223>  Chimeric

<400>  7

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asn Ile Tyr Ser Tyr
            20                  25                  30

Phe Ser Trp Tyr Gln Gln Lys Pro Gly Lys Ala Pro Lys Leu Leu Ile
        35                  40                  45

Tyr Thr Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Val Thr Pro Tyr
                85                  90                  95

Thr Phe Gly Gly Gly Thr Lys Val Glu Ile Lys
            100                 105

<210>  8
<211>  123
<212>  PRT
<213>  Artificial

<220>
<223>  Chimeric

<400>  8

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr His Tyr
            20                  25                  30

Gly Met Asn Trp Val Arg Gln Ala Pro Gly Gln Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Arg Arg Arg Tyr Glu Gly Asn Tyr Val Phe Tyr Tyr Phe Asp Tyr
            100                 105                 110

Trp Gly Gln Gly Thr Thr Val Thr Val Ser Ser
        115                 120


<210>  9
<211>  107
<212>  PRT
<213>  Artificial

<220>
<223>  Chimeric

<400>  9

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asn Ile Tyr Ser Tyr
            20                  25                  30

Phe Ser Trp Tyr Gln Gln Lys Pro Gly Lys Ala Pro Lys Leu Leu Ile
        35                  40                  45

Tyr Thr Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Val Thr Pro Tyr
                85                  90                  95

Thr Phe Gly Gly Gly Thr Lys Val Glu Ile Lys
            100                 105

<210>  10
<211>  123
<212>  PRT
<213>  Artificial

<220>
<223>  Chimeric

<400>  10

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr His Tyr
            20                  25                  30

Gly Met Asn Trp Val Arg Gln Ala Pro Gly Lys Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Arg Arg Arg Tyr Glu Gly Asn Tyr Val Phe Tyr Tyr Phe Asp Tyr
            100                 105                 110

Trp Gly Gln Gly Thr Thr Val Thr Val Ser Ser
        115                 120

<210>  11
<211>  107
<212>  PRT
<213>  Artificial

<220>
<223>  Chimeric

<400>  11

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asn Ile Tyr Ser Tyr
            20                  25                  30

Phe Ser Trp Tyr Gln Gln Lys Pro Gly Lys Ala Pro Lys Leu Leu Ile
        35                  40                  45

Tyr Thr Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Val Thr Pro Tyr
                85                  90                  95

Thr Phe Gly Gly Gly Thr Lys Val Glu Ile Lys
            100                 105


<210>  12
<211>  123
<212>  PRT
<213>  Artificial

<220>
<223>  Chimeric

<400>  12

Gln Ile Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr His Tyr
            20                  25                  30

Gly Met Asn Trp Val Arg Gln Ala Pro Gly Lys Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Val Phe Ser Leu Asp Ala Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Arg Arg Arg Tyr Glu Gly Asn Tyr Val Phe Tyr Tyr Phe Asp Tyr
            100                 105                 110

Trp Gly Gln Gly Thr Thr Val Thr Val Ser Ser
        115                 120

<210>  13
<211>  107
<212>  PRT
<213>  Artificial

<220>
<223>  Chimeric

<400>  13

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asn Ile Tyr Ser Tyr
            20                  25                  30

Phe Ser Trp Tyr Gln Gln Lys Pro Gly Lys Ala Pro Lys Leu Leu Ile
        35                  40                  45

Tyr Thr Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Val Thr Pro Tyr
                85                  90                  95

Thr Phe Gly Gly Gly Thr Lys Val Glu Ile Lys
            100                 105

<210>  14
<211>  123
<212>  PRT
<213>  Artificial

<220>
<223>  Chimeric

<400>  14

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr His Tyr
            20                  25                  30

Gly Met Asn Trp Val Arg Gln Ala Pro Gly Gln Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Arg Arg Arg Tyr Glu Gly Asn Tyr Val Phe Tyr Tyr Phe Asp Tyr
            100                 105                 110

Trp Gly Gln Gly Thr Thr Val Thr Val Ser Ser
        115                 120

<210> 15
<211> 107
<212> PRT
<213> Artificial

<220>
<223> Chimeric

<400> 15

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asn Ile Tyr Ser Tyr
            20                  25                  30

Phe Ser Trp Tyr Gln Gln Lys Pro Gly Lys Ala Pro Lys Leu Leu Val
        35                  40                  45

Tyr Thr Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Val Thr Pro Tyr
                85                  90                  95

Thr Phe Gly Gly Gly Thr Lys Val Glu Ile Lys
            100                 105

<210>  16
<211>  123
<212>  PRT
<213>  Artificial

<220>
<223>  Chimeric

<400>  16

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr His Tyr
            20                  25                  30

Gly Met Asn Trp Val Arg Gln Ala Pro Gly Lys Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Arg Arg Arg Tyr Glu Gly Asn Tyr Val Phe Tyr Tyr Phe Asp Tyr
            100                 105                 110

Trp Gly Gln Gly Thr Thr Val Thr Val Ser Ser
        115                 120


<210>  17
<211>  107
<212>  PRT
<213>  Artificial

<220>
<223>  Chimeric

<400>  17

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asn Ile Tyr Ser Tyr
            20                  25                  30

Phe Ser Trp Tyr Gln Gln Lys Pro Gly Lys Ala Pro Lys Leu Leu Val
        35                  40                  45

Tyr Thr Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Val Thr Pro Tyr
                85                  90                  95

Thr Phe Gly Gly Gly Thr Lys Val Glu Ile Lys
            100                 105

<210>  18
<211>  123
<212>  PRT
<213>  Artificial

<220>
<223>  Chimeric

<400>  18

Gln Ile Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr His Tyr
            20                  25                  30

Gly Met Asn Trp Val Arg Gln Ala Pro Gly Lys Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Val Phe Ser Leu Asp Ala Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Arg Arg Arg Tyr Glu Gly Asn Tyr Val Phe Tyr Tyr Phe Asp Tyr
            100                 105                 110

Trp Gly Gln Gly Thr Thr Val Thr Val Ser Ser
        115                 120

<210>  19
<211>  107
<212>  PRT

<213>  Artificial

<220>
<223>  Chimeric

<400>  19

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asn Ile Tyr Ser Tyr
            20                  25                  30

Phe Ser Trp Tyr Gln Gln Lys Pro Gly Lys Ala Pro Lys Leu Leu Val
        35                  40                  45

Tyr Thr Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Val Thr Pro Tyr
                85                  90                  95

Thr Phe Gly Gly Gly Thr Lys Val Glu Ile Lys
            100                 105

<210>  20
<211>  5
<212>  PRT
<213>  Mus musculus

<400>  20

His Tyr Gly Met Asn
1               5

<210>  21
<211>  17
<212>  PRT
<213>  Mus musculus

<400>  21

Trp Ile Asn Thr Tyr Thr Gly Glu Pro Thr Tyr Ala Asp Asp Phe Lys
1               5                   10                  15

Gly

<210>  22
<211>  14

<212>  PRT
<213>  Mus musculus

<400>  22

Arg Arg Tyr Glu Gly Asn Tyr Val Phe Tyr Tyr Phe Asp Tyr
1               5                   10


<210>  23
<211>  11
<212>  PRT
<213>  Mus musculus

<400>  23

Arg Ala Ser Glu Asn Ile Tyr Ser Tyr Phe Ser
1               5                   10


<210>  24
<211>  7
<212>  PRT
<213>  Mus musculus

<400>  24

Thr Ala Lys Thr Leu Ala Glu
1               5


<210>  25
<211>  9
<212>  PRT
<213>  Mus musculus

<400>  25

Gln His His Tyr Val Thr Pro Tyr Thr
1               5


<210>  26
<211>  453
<212>  PRT
<213>  Artificial

<220>
<223>  Chimeric

<400>  26

Gln Val Gln Leu Val Gln Ser Gly Ser Glu Leu Lys Lys Pro Gly Ala
1               5                   10                  15


Ser Val Lys Val Ser Cys Lys Ala Ser Gly Tyr Thr Phe Thr His Tyr
            20                  25                  30


Gly Met Asn Trp Val Arg Gln Ala Pro Gly Gln Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Arg Arg Arg Tyr Glu Gly Asn Tyr Val Phe Tyr Tyr Phe Asp Tyr
            100                 105                 110

Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Arg Val Glu Pro Lys
    210                 215                 220

Ser Cys Asp Lys Thr His Thr Cys Pro Pro Cys Pro Ala Pro Glu Phe
225                 230                 235                 240

Glu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Ser Ile Glu Lys Thr Ile Ser Lys Ala Lys Gly Gln Pro Arg Glu Pro
            340                 345                 350

Gln Val Tyr Thr Leu Pro Pro Ser Arg Glu Glu Met Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450

<210>  27
<211>  453
<212>  PRT
<213>  Artificial

<220>
<223>  Chimeric

<400>  27

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr His Tyr
            20                  25                  30

Gly Met Asn Trp Val Arg Gln Ala Pro Gly Gln Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Arg Arg Arg Tyr Glu Gly Asn Tyr Val Phe Tyr Tyr Phe Asp Tyr
            100                 105                 110

Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Arg Val Glu Pro Lys
    210                 215                 220

Ser Cys Asp Lys Thr His Thr Cys Pro Pro Cys Pro Ala Pro Glu Ala
225                 230                 235                 240

Glu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Ser Ile Glu Lys Thr Ile Ser Lys Ala Lys Gly Gln Pro Arg Glu Pro
            340                 345                 350

Gln Val Tyr Thr Leu Pro Pro Ser Arg Glu Glu Met Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450

<210>  28
<211>  453
<212>  PRT
<213>  Artificial

<220>
<223>  Chimeric

<400>  28

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr His Tyr
            20                  25                  30

Gly Met Asn Trp Val Arg Gln Ala Pro Gly Gln Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Arg Arg Arg Tyr Glu Gly Asn Tyr Val Phe Tyr Tyr Phe Asp Tyr
            100                 105                 110

Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Arg Val Glu Pro Lys
    210                 215                 220

Ser Cys Asp Lys Thr His Thr Cys Pro Pro Cys Pro Ala Pro Glu Ala
225                 230                 235                 240

Glu Gly Ala Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ser
                325                 330                 335

Ser Ile Glu Lys Thr Ile Ser Lys Ala Lys Gly Gln Pro Arg Glu Pro
            340                 345                 350

Gln Val Tyr Thr Leu Pro Pro Ser Arg Glu Glu Met Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450

<210>  29
<211>  453
<212>  PRT
<213>  Artificial

<220>
<223>  Chimeric

<400>  29

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr His Tyr
            20                  25                  30

Gly Met Asn Trp Val Arg Gln Ala Pro Gly Gln Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Arg Arg Arg Tyr Glu Gly Asn Tyr Val Phe Tyr Tyr Phe Asp Tyr
            100                 105                 110

Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Arg Val Glu Pro Lys
    210                 215                 220

Ser Cys Asp Lys Thr His Thr Cys Pro Pro Cys Pro Ala Pro Glu Ala
225                 230                 235                 240

Glu Gly Ala Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Ser Ile Glu Lys Thr Ile Ser Lys Ala Lys Gly Gln Pro Arg Glu Pro
            340                 345                 350

Gln Val Tyr Thr Leu Pro Pro Ser Arg Glu Glu Met Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450

<210>  30
<211>  330
<212>  PRT
<213>  Artificial

<220>
<223>  Homo sapiens modified

<400>  30

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Ala Glu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Ser Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  31
<211>  330
<212>  PRT
<213>  Artificial

<220>
<223>  Homo sapiens modified

<400>  31

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Phe Glu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Ser Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210> 32
<211> 330
<212> PRT
<213> Artificial

<220>
<223> Homo sapiens modified

<400> 32

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Ala Glu Gly Ala Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ser Ser Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  33
<211>  330
<212>  PRT
<213>  Artificial

<220>
<223>  Homo sapiens modified

<400>  33

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Ala Glu Gly Ala Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Ser Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  34
<211>  37
<212>  PRT
<213>  Artificial

<220>
<223>  Chimeric

<400>  34

Phe Thr His Tyr Gly Met Asn Trp Val Arg Gln Ala Pro Gly Gln Gly
1               5                   10                  15

Leu Glu Trp Met Gly Trp Ile Asn Thr Tyr Thr Gly Glu Pro Thr Tyr
            20                  25                  30

Ala Asp Asp Phe Lys
        35

<210>  35

<211>  37
<212>  PRT
<213>  artificial

<220>
<223>  Chimeric

<400>  35

Phe Thr His Tyr Gly Met Asn Trp Val Arg Gln Ala Pro Gly Gln Gly
1               5                   10                  15

Leu Lys Trp Met Gly Trp Ile Asn Thr Tyr Thr Gly Glu Pro Thr Tyr
            20                  25                  30

Ala Asp Asp Phe Lys
        35

## Description

### CROSS-REFERENCE TO RELATED APPLICATIONS

This application claims the benefit of U.S. Provisional Application Nos. US 62/307,606 filed 14 March 2016 and US 62/404,780 filed 6 October 2016, the disclosures of which are incorporated herein by reference in their entireties, including any drawings.

### REFERENCE TO SEQUENCE LISTING

The present application is being filed along with a Sequence Listing in electronic format. The Sequence Listing is provided as a file entitled "CD39-3_ST25", created 13 March 2017, which is 64 KB in size. The information in the electronic format of the Sequence Listing is incorporated herein by reference in its entirety.

### FIELD OF THE INVENTION

The present invention relates to antigen-binding compounds (e.g., antibodies) that inhibit CD39. The invention also relates to cells producing such compounds; methods of making such compounds, and antibodies, fragments, variants, and derivatives thereof; pharmaceutical compositions comprising the same; methods of using the compounds to diagnose, treat or prevent diseases, e.g., cancer.

### BACKGROUND

Eight different ENTPD genes encode members of the NTPDase protein family. The individual NTPDase subtypes differ in cellular location and functional properties. Plasma membrane-bound nucleoside triphosphate diphosphohydrolases control nucleotide levels at the cell surface by hydrolyzing the c and b phosphates of nucleotides.

NTPDase 1 (ectonucleoside triphosphate diphosphohydrolase1), also known as CD39/ENTPD1 or vascular CD39, functions together with another enzyme, CD73 (ecto-5'-nucleotidase), to hydrolyze extracellular adenosine triphosphate (ATP) and adenosine diphosphate (ADP) to generate adenosine, which binds to adenosine receptors and inhibits T-cell and natural killer (NK)-cell responses, thereby suppressing the immune system. The generation of adenosine via the CD73/CD39 pathway is recognized as a major mechanism of regulatory T cell (Treg) immunosuppressive function. The number of CD39⁺ Tregs is increased in some human cancers, and the importance of CD39⁺ Tregs in promoting tumor growth and metastasis has been demonstrated using several in vivo models. However, CD39 is also expressed by tumor cells and CD39⁺ tumor cells can mediate immunosuppression via the adenosine pathway. CD39 in cancer cells displays ATPase activity and, together with CD73, generates adenosine. CD73⁺CD39⁺ cancer cells inhibited the proliferation of CD4 and CD8 T cells and the generation of cytotoxic effector CD8 T cells (CTL) in a CD39- and adenosine-dependent manner. Antibodies that bind and inhibit CD39 are disclosed in WO2009/095478. Hayes et al. (2015) Am. J. Transl. Res. 7(6):1181-1188 makes use of an anti-CD39 that binds FcyR and has effector function but it stated to also be blocking.

Consequently, CD39 expression on different cell types, including immune cells and tumor cells, combined with use of antibodies that either do not actually block CD39 or are not pure blockers, create a complex setting for evaluation of the underlying activity of antibodies.

### SUMMARY OF THE INVENTION

In one aspect, provided are antibodies that bind an epitope present on human CD39 expressed at the surface of cells, including tumor cells and leukocytes, and that potently inhibit the enzymatic (ATPase activity) activity of the CD39 enzyme.

In one embodiment, provided is an anti-CD39 antigen binding domain, or a protein that comprises the antigen binding domain (e.g., a monoclonal antibody, a multispecific binding protein, a bispecific antibody, etc.), the antigen binding domain comprising a heavy chain variable region (VH) comprising CDR1, CDR2 and CDR3 having the respective amino acid sequences shown in SEQ ID NOS: 20, 21 and 22 and a light chain variable region (VL) CDR1, CDR2 and CDR3 having the respective amino acid sequences shown in SEQ ID NOS: 23, 24 and 25, wherein the (VH) comprises an amino acid sequence at least 90%, 95% or 98% identical to the amino acid sequence of SEQ ID NO: 6, and a light chain variable region (VL) comprising an amino acid sequence at least 90%, 95% or 98% identical to the amino acid sequence of SEQ ID NO: 7.

In one embodiment, the VH comprises a human VH acceptor framework and the VL comprises a human VL acceptor framework. In one embodiment, the VH segment of the VH human acceptor framework is from IGHV7-4-1 and the J-segment is from IGHJ6. In one embodiment, the VH human acceptor framework is from IGHV7-4-1*01. In one embodiment, the CDR1, 2 and 3 of the VH are from the murine IGHV9-3-1 gene, optionally from IGHV9-3-1*01. In one embodiment, the VL domain human acceptor framework is from IGKJ4 or IGKJ2, optionally the VL domain human acceptor framework is from IGKJ4*01. In one embodiment, the CDR1, 2 and 3 of the VL are from the murine IGKV12-44 gene, optionally from IGKV12-44*01. In one embodiment, the human heavy chain acceptor framework comprises a lysine at Abnum position 46 and an aspartic acid at Abnum position 62 (CDR2), and contains no other framework mutations compared to a naturally occurring human VH segment. In one embodiment, the human light chain acceptor framework is free of mutations compared to a naturally occurring human VH segment. In one embodiment, the human heavy chain acceptor framework comprises a glutamic acid at Abnum position 46 (FR2) and an aspartic acid at Abnum position 62 (CDR2) and contains no framework mutations compared to a naturally occurring human VH segment. In one embodiment, the human light chain acceptor framework is free of mutations compared to a naturally occurring human VH segment.

In one embodiment, the VH comprises a lysine at Abnum position 46 and an aspartic acid at Abnum position 62 (CDR2), and contains no other framework mutations compared to a naturally occurring human VH segment. In one embodiment, a VH comprises frameworks that are free of mutations compared to a naturally occurring human VH segment. In one embodiment, the VH comprises a glutamic acid at Abnum position 46 (FR2) and an aspartic acid at Abnum position 62 (CDR2) and contains no other framework mutations compared to a naturally occurring human VH segment, and the VL comprises frameworks that are free of mutations compared to a naturally occurring human VL segment.

In one embodiment, the VH comprises a FR2-CDR2 segment comprising the amino acid sequence of SEQ ID NO: 35.

In one embodiment of any aspect herein, the VH comprises the heavy chain CDR1, CDR2 and CDR3 having the respective amino acid sequences shown in SEQ ID NOS: 20, 21 and 22. In one embodiment, the VL comprises the light chain CDR1, CDR2 and CDR3 having the respective amino acid sequences shown in SEQ ID NOS: 23, 24 and 25.

In one embodiment, provided is an anti-CD39 antigen binding domain, or a protein that comprises the antigen binding domain (e.g., a monoclonal antibody, a multispecific binding protein, a bispecific antibody, etc.), the antigen binding domain comprising:
(a) a heavy chain variable region comprising an amino acid sequence of SEQ ID NO: 6, optionally further comprising one, two or three amino acid residue substitutions in a framework region, and
(b) a light chain variable region comprising an amino acid sequence of SEQ ID NO: 7, optionally further comprising one, two or three amino acid residue substitutions in a framework region.

In one aspect of any of the embodiments, the heavy chain variable region comprises a glutamic acid (E) residue at position 46 (Abnum numbering).

In another aspect of any of the embodiments, the heavy chain variable region comprises a E46K substitution (Abnum numbering).

In one embodiment, provided is an anti-CD39 antigen binding domain, or a protein that comprises the antigen binding domain (e.g., a monoclonal antibody, a multispecific binding protein, a bispecific antibody, etc.), the antigen binding domain selected from the group consisting of:
(a) an antibody binding domain comprising a heavy chain variable region comprising an amino acid sequence of SEQ ID NO: 6 and a light chain variable region comprising an amino acid sequence of SEQ ID NO: 7;
(b) an antibody binding domain comprising a heavy chain variable region comprising an amino acid sequence of SEQ ID NO: 8 and a light chain variable region comprising an amino acid sequence of SEQ ID NO: 9;
(c) an antibody binding domain comprising a heavy chain variable region comprising an amino acid sequence of SEQ ID NO: 10 and a light chain variable region comprising an amino acid sequence of SEQ ID NO: 11;
(d) an antibody binding domain comprising a heavy chain variable region comprising an amino acid sequence of SEQ ID NO: 12 and a light chain variable region comprising an amino acid sequence of SEQ ID NO: 13;
(e) an antibody binding domain comprising a heavy chain variable region comprising an amino acid sequence of SEQ ID NO: 14 and a light chain variable region comprising an amino acid sequence of SEQ ID NO: 15;
(f) an antibody binding domain comprising a heavy chain variable region comprising an amino acid sequence of SEQ ID NO: 16 and a light chain variable region comprising an amino acid sequence of SEQ ID NO: 17; and
(g) an antibody binding domain comprising a heavy chain variable region comprising an amino acid sequence of SEQ ID NO: 18 and a light chain variable region comprising an amino acid sequence of SEQ ID NO: 19.

In one embodiment, provided is a monoclonal antibody that binds human CD39 and that neutralizes the ATPase activity of CD39, selected from the group consisting of:
(a) an antibody comprising a heavy chain variable region comprising an amino acid sequence of SEQ ID NO: 6 and a light chain variable region comprising an amino acid sequence of SEQ ID NO: 7;
(b) an antibody comprising a heavy chain variable region comprising an amino acid sequence of SEQ ID NO: 8 and a light chain variable region comprising an amino acid sequence of SEQ ID NO: 9;
(c) an antibody comprising a heavy chain variable region comprising an amino acid sequence of SEQ ID NO: 10 and a light chain variable region comprising an amino acid sequence of SEQ ID NO: 11;
(d) an antibody comprising a heavy chain variable region comprising an amino acid sequence of SEQ ID NO: 12 and a light chain variable region comprising an amino acid sequence of SEQ ID NO: 13;
(e) an antibody comprising a heavy chain variable region comprising an amino acid sequence of SEQ ID NO: 14 and a light chain variable region comprising an amino acid sequence of SEQ ID NO: 15;
(f) an antibody comprising a heavy chain variable region comprising an amino acid sequence of SEQ ID NO: 16 and a light chain variable region comprising an amino acid sequence of SEQ ID NO: 17; and
(g) an antibody comprising a heavy chain variable region comprising an amino acid sequence of SEQ ID NO: 18 and a light chain variable region comprising an amino acid sequence of SEQ ID NO: 19.

In one aspect, provided are anti-CD39 antibodies with human frameworks that have modified salt bridges in the heavy chain FR2 (compared to an antibody having murine frameworks). Salt bridges in proteins are bonds between oppositely charged residues that are sufficiently close to each other to experience electrostatic attraction.

In one aspect, provided are anti-CD39 antibodies with human frameworks that both have high affinity antigen binding, ability to neutralize the ATPase activity of CD39 and physicochemical stability, e.g., in pharmaceutical formulations. In certain embodiments, disclosed are antibodies that bind human CD39 with human heavy and light chain variable region frameworks, wherein the heavy chain framework 2 (FR2) is of IgHV7-4-1 origin and comprises a lysine or a glutamic acid residue at position 46 (Abnum numbering), and wherein the heavy chain CDR2 is of murine IgHV9 origin and comprises an aspartic acid residue at position 62 (Abnum numbering).

The antibodies of the disclosure can bind a human CD39 polypeptide with high affinity and neutralize the enzymatic activity of membrane-bound CD39 protein expressed at the surface of cells, and, moreover, without substantially inducing or increasing intracellular internalization of, or more generally down-modulation of, cell surface-expressed CD39. The antibodies therefore do not depend on CD39 down-modulation or depletion of CD39-expressing cells for their CD39 inhibitory activity. Advantageously, these antibodies can be used as "pure" CD39 blockers, targeted to vascular CD39, permitting immunomodulatory activity without unwanted toxicity at CD39-expressing vascular cells. The antibodies inhibit the enzymatic activity of membrane-bound CD39 protein expressed at the surface of cells without the need to substantially bind Fcγ receptors and/or without substantially directing ADCC and/or CDC toward a CD39-expressing cell. Optionally, the antibodies retain an Fc domain and retain binding to human FcRn.

In one aspect of any embodiment herein, a heavy chain variable region is fused to human heavy chain CH1 constant domain, a human hinge domain, and a human Fc domain. In one embodiment, and a light chain variable region is fused to human light chain (CK) constant domain.

In one aspect of any embodiment herein, an antibody may comprise a human Fc domain that is modified to have decreased or substantially lack binding to human CD16, CD32a, CD32b and CD64. When the antibody functions in a setting characterized by absence of Fcγ receptor-expressing cells the antibody does not induce or increase CD39 down-modulation on cells, despite retaining the ability to bind CD39 polypeptides in bivalent manner. Decreased or substantially lack binding to human CD16, CD32a, CD32b and CD64 eliminates potential induction of CD39 down-modulation in vivo (in the presence of Fcγ receptor-expressing cells). The property of non-internalization and non-down-modulation can confer an improved pharmacology in vivo, in turn leading to a more complete neutralization of CD39 activity in vivo.

In one embodiment, a non-depleting antibody will provide an at least 70%, 80%, 90% reduction in the ATPase activity of a CD39-expressing cell (e.g., as assessed by decrease in AMP generation by a CD39+ cell such as a B cell, a Ramos cell, as measured by mass spectrometry), at a concentration compatible with administration of an antibody to a human. The antibodies identified by these methods were then tested in cellular enzymatic activity assays using purified antibody, and found to strongly neutralize the enzymatic activity of vascular human CD39. The epitope on CD39 bound by these antibodies is present on CD39 polypeptides as expressed by a range of cells, e.g., cancer cells, CD4 T cells, CD8 T cells, B cells, transfected cells, and binds with high affinity as determined by flow cytometry. For example, an antibody can be characterized by an EC₅₀, as determined by flow cytometry, of no more than 2 µg/ml, no more than 1 µg/ml, no more than 0.5 µg/ml, no more than 0.1 µg/ml or no more than 0.05 µg/ml, for binding to cells that express at their surface a CD39 polypeptide. In one embodiment the cells are cells that are made to express CD39 at their surface. In one embodiment the cells are cells that endogenously express CD39 at their surface, e.g., regulatory T (TReg) cells, B cells, cancer cells, leukemia cells, bladder cancer cells, glioma cells, glioblastoma cells, ovarian cancer cells, melanoma cells, prostate cancer cells, thyroid cancer cells, esophageal cancer cells or breast cancer cells.

Optionally, the CD39-binding agent binds to cells expressing at their surface human non-human primate CD39 polypeptide (e.g., a cynomolgus monkey CD39 polypeptide).

In one aspect, provided is an anti-CD39 antibody capable of specifically inhibiting the enzymatic activity of membrane-bound CD39 protein (vascular CD39; the polypeptide of SEQ ID NO: 1) expressed at the surface of cells without substantially binding to human Fcγ receptors (e.g., CD16) and/or C1q, and/or without substantially directing ADCC and/or CDC toward a CD39-expressing cell.

In one aspect, provided is an anti-CD39 antibody capable of inhibiting the enzymatic activity of membrane-bound vascular CD39 protein (comprising an amino acid sequence of SEQ ID NO: 1) expressed at the surface of cells without substantially causing the down-modulation (e.g., internalization) of cell surface-expressed CD39. In one embodiment, the antibodies do not substantially bind (e.g., via their Fc domain) to human Fcγ receptors (e.g., CD16, CD32a, CD32b, CD64) and/or C1q, and/or do not substantially directing ADCC and/or CDC toward a CD39-expressing cell. Optionally, the antibodies retain an Fc domain and retain binding to human FcRn.

In one embodiment, the antibodies are used as pure blockers and administered in an amount effective to neutralize the enzymatic activity of CD39 for a desired period of time, e.g., 1 week, 2 weeks, a month, until the next successive administration of anti-CD39 antibody.

In one embodiment, the antibodies are administered at a dosage and/or frequency that provides a blood concentration of antibody equal to at least the EC₅₀, EC₇₀ or EC₁₀₀ for inhibition of ATPase activity, optionally wherein the concentration is maintained for at least 1 week, 2 weeks, a month, or until the next successive administration of the anti-CD39 antibody. In one embodiment, the blood concentration is greater than the respective EC₅₀, EC₇₀ or EC₁₀₀ for ADCC activity towards CD39-expressing cells by an equivalent antibody that has an Fc domain that mediates CD16 binding, e.g., IgG1 (e.g., tumor cells, TReg cells and/or B cells).

Optionally the antibodies are pure blockers and directed to neutralize the enzymatic activity of CD39 in the tumor environment, without the ability to mediate effector functions, e.g., ADCC, CDC, or FcγR-mediated crosslinking of CD39. Optionally the antibodies comprise a modified Fc domain, e.g., to decrease protease sensitivity (e.g., toward proteases such as MMPs in the tumor environment) and/or to decrease binding to human Fcγ receptors (e.g., CD16).

In any embodiment herein, an antibody may comprise a heavy chain comprising an amino acid sequence of SEQ ID NOS: 26, 27, 28 or 29. In one embodiment, provided is an antibody comprising a heavy chain comprising an amino acid sequence of SEQ ID NOS: 26, 27, 28 or 29, and a light chain comprising an amino acid sequence at least 90%, 95% or 98% identical to the amino acid sequence of SEQ ID NO: 7. In one embodiment, provided is an antibody comprising a heavy chain comprising an amino acid sequence of SEQ ID NOS: 26, 27, 28 or 29, and a light chain comprising an amino acid sequence of SEQ ID NO: 7. In one embodiment, provided is an antibody comprising a heavy chain comprising an amino acid sequence of SEQ ID NOS: 26, 27, 28 or 29, and a light chain comprising an amino acid sequence of SEQ ID NO: 9.

In one embodiment, provided is an antibody that binds human CD39 and that neutralizes the ATPase activity thereof, selected from the group consisting of:
(a) an antibody comprising a heavy chain comprising an amino acid sequence of SEQ ID NO: 26 and a light chain comprising an amino acid sequence of SEQ ID NO: 9;
(b) an antibody comprising a heavy chain comprising an amino acid sequence of SEQ ID NO: 27 and a light chain comprising an amino acid sequence of SEQ ID NO: 9;
(c) an antibody comprising a heavy chain comprising an amino acid sequence of SEQ ID NO: 28 and a light chain comprising an amino acid sequence of SEQ ID NO: 9; and
(d) an antibody comprising a heavy chain comprising an amino acid sequence of SEQ ID NO: 29 and a light chain comprising an amino acid sequence of SEQ ID NO: 9.

In one embodiment, the CD39 neutralizing antibodies can be characterized by being capable of causing a decrease in cells' ATPase activity of CD39, optionally causing a decrease of AMP generation by a CD39-expressing cell, by at least 70%, 80% or 90%. In one embodiment, the CD39-neutralizing antibodies can be characterized by an EC₅₀ for inhibition of ATPase activity (e.g., EC₅₀ for inhibition of AMP generation by a CD39-expressing cell) of CD39 expressed by a cell of no more than 1 µg/ml, optionally no more than 0.5 µg/ml, optionally no more than 0.2 µg/ml.

Optionally, inhibition of ATPase activity of CD39 expressed by a cell is determined by assessing neutralization of ATPase activity in Ramos cells by quantifying hydrolysis of ATP.

In one aspect, neutralization of the ATPase activity is determined by bringing CD39-expressing cells (e.g., Ramos lymphoma cells as used herein, available for example from the ATCC, reference CRL-1596) into contact with an antibody, and assessing production of AMP, e.g., by mass spectrometry, wherein a decrease in AMP generated indicates neutralization of ATPase activity. Optionally an antibody causes a decrease of AMP generated by at least 70%, 80% or 90% in this assay.

The antibodies will be useful in inhibiting CD39-mediated ATP hydrolysis, e.g., thereby leading to a decrease in the concentration of adenosine in the tumor microenvironment. These antibodies will therefore be useful in reversing the immunosuppressive effect of CD39 and/or adenosine on T cells, B cells and other cells that express adenosine receptors (A2A receptors), for example in the treatment of cancer. In one embodiment, the anti-CD39 antibody neutralizes adenosine-mediated inhibition of proliferation, cytokine production, cytotoxicity and/or NFκB activity in T cells.

The antibodies will be useful in inhibiting the production, amounts and/or concentrations of adenosine into the tumor microenvironment.

Provided is a method for treating an individual, the method comprising administering to an individual (e.g., an individual having a disease, a tumor, etc.) a therapeutically active amount of any of the anti-CD39 antigen binding compounds described herein. In one aspect provided is a method for treating an individual, the method comprising, consisting essentially of or consisting of: administering to an individual (e.g., an individual having a disease, a tumor, etc.) a therapeutically active amount of an antigen binding compound of the disclosure that inhibits a CD39 polypeptide. In one embodiment, the antibody inhibits a CD39 polypeptide in a cellular assay. The compound is in one embodiment a non-depleting antibody (an antibody that does not deplete cells to which it binds, e.g., an Fc silent antibody). Optionally, the compound binds to CD39 polypeptides in bivalent manner. Optionally, the antibody is a chimeric, humanized or human antibody. Optionally, the antibody is a bispecific antibody. Optionally, the antibody comprises a heavy chain constant region of IgG1 or IgG4 isotype.

In one aspect provided is a method for decreasing ATP hydrolysis by a CD39-expressing cell (e.g., an immune cell and/or a tumor cell in an individual), or a method for neutralizing of the enzymatic activity of cellular CD39, the method comprising, consisting essentially of or consisting of: bringing the CD39-expressing cell into contact with an antibody of the disclosure that inhibits CD39. In one embodiment, the step of bringing the CD39-expressing cell into contact with an antigen binding compound of the disclosure comprises administering to an individual a therapeutically active amount of an antibody that inhibits CD39. In one embodiment the individual has a cancer.

In one aspect provided is a method for decreasing adenosine present in the tumor environment (e.g., in an individual), the method comprising, consisting essentially of or consisting of: administering to an individual a therapeutically active amount of an antibody of the disclosure that neutralizes the activity of a CD39 polypeptide. In one embodiment the individual has a cancer.

In one embodiment, the active amount of an antibody that inhibits a CD39 polypeptide is an amount effective to achieve and/or maintain (e.g., until the subsequent administration of antigen binding compound) a blood concentration of at least the EC₅₀, optionally the EC₇₀, optionally substantially the EC₁₀₀, for inhibition of CD39-mediated catabolism of ATP to AMP in an individual. In one embodiment, the active amount of an antigen binding compound that inhibits a CD39 polypeptide is an amount effective to achieve the EC₅₀, optionally the EC₇₀, optionally substantially the EC₁₀₀, for inhibition of CD39-mediated catabolism of ATP to AMP in an extravascular tissue of an individual. In one embodiment, the active amount an antigen binding compound that inhibits a CD39 polypeptide is an amount effective to achieve the EC₅₀, optionally the EC₇₀, optionally substantially the EC₁₀₀, for inhibition of CD39-mediated catabolism of ATP to AMP in an individual. In one embodiment, the active amount of an antigen binding compound that inhibits a CD39 polypeptide is between 1 and 20 mg/kg body weight. In one embodiment, the active amount is administered to an individual weekly, every two weeks, monthly or every two months.

Optionally the individual is a human having or who is susceptible to having a cancer. Optionally the individual is a human having or who is susceptible to having a cancer characterized by malignant cells that express vascular CD39. Optionally the individual is a human having or who is susceptible to having a cancer and who has detectable levels of circulating or tumor-infiltrating leukocytes that express vascular CD39.

The antibodies are optionally characterized by binding affinity (K_{D}) for a human CD39 polypeptide of less than (better than) 10⁻⁹ M, preferably less than 10⁻¹⁰ M, or preferably less than 10⁻¹¹, and/or by binding human CD39 with an EC₅₀ lower than (better binding than) 1 µg/ml, preferably wherein the antibody has an EC₅₀ of no more than 0.5 µg/ml, optionally no more than 0.2 µg/ml, optionally no more than 0.1 µg/ml, for binding to cells (e.g., tumor cells, Ramos tumor cells (ATCC accession number CRL-1596) expressing human CD39 at the cell surface (e.g. as determined by flow cytometry).

The antibodies are optionally chimeric, human or humanized antibodies.

The antibodies are optionally characterized by an EC₅₀ for neutralization of the enzymatic activity of CD39 in CD39-expressing cells of less than (better than) 1 µg/ml, optionally less than 0.5 µg/ml.

In one embodiment, the antibody is a monoclonal antibody or a fragment thereof that retains binding specificity and ability to neutralize the enzymatic activity of CD39. In one embodiment, the antibody is an antibody comprising an Fc domain of any human IgG isotype (e.g., IgG1, IgG2, IgG3, or IgG4) modified to reduce binding between the Fc domain and an Fcγ receptor (e.g., CD16). Optionally, the antigen-binding compound does not comprise an Fc domain capable of inducing antibody mediated cellular cytotoxicity (ADCC) and/or CDC; optionally the antigen-binding compound does not comprise an Fc domain capable of substantially binding to a FcγRIIIA (CD16) polypeptide (e.g., comprises an Fc domain not capable of substantially binding to a FcγRIIIA (CD16) polypeptide; lacks an Fc domain (e.g., lacks a CH2 and/or CH3 domain). In one embodiment, the Fc domain (e.g., of human IgG1, IgG2, IgG3 or IgG4 isotype) comprises an amino acid modification (e.g., substitution) compared to a wild-type Fc domain, wherein the substitution reduces the ability of the Fc domain (or antibodies containing it) to bind to an Fcγ receptor (e.g., CD16) and/or to bind complement.

Also provided are nucleic acids encoding the human or humanized antibody or antibody fragment having any of the foregoing properties, a vector comprising such a nucleic acid, a cell comprising such a vector, and a method of producing a human anti-CD39 antibody, comprising culturing such a cell under conditions suitable for expression of the anti-CD39 antibody. The disclosure also relates to compositions, such as pharmaceutically acceptable compositions and kits, comprising such proteins, nucleic acids, vectors, and/or cells and typically one or more additional ingredients that can be active ingredients or inactive ingredients that promote formulation, delivery, stability, or other characteristics of the composition (e.g., various carriers). The disclosure further relates to various new and useful methods making and using such antibodies, nucleic acids, vectors, cells, organisms, and/or compositions, such as in the modulation of CD39-mediated biological activities, for example in the treatment of diseases related thereto, notably cancers.

The disclosure also provides a method of potentiating the activity of lymphocytes (e.g., T cells) in a subject in need thereof, or for restoring the activity of lymphocytes (e.g., T cells), or a method of relieving the adenosine-mediated inhibition of lymphocytes (e.g., T cells), which method comprises administering to the subject an effective amount of any of the foregoing compositions. In one embodiment, the subject is a patient suffering from cancer. For example, the patient may be suffering from a solid tumor, e.g., colorectal cancer, renal cancer, ovarian cancer, lung cancer, breast cancer or malignant melanoma. Alternatively, the patient may be suffering from a hematopoietic cancer, e.g., acute myeloid leukaemia, chronic myeloid leukaemia, multiple myeloma, or non-Hodgkin's lymphoma.

The disclosure also provides a method for treatment of disease in an individual, the treatment comprising administering to the individual an anti-CD39 antibody that neutralizes the enzymatic activity of CD39 for at least one administration cycle in which the anti-CD39 antibody is administered at least once, optionally at least twice, in an amount effective to achieve, and/or to maintain between two successive administrations of the anti-CD39 antibody, a concentration in blood (serum) or an extravascular tissue (e.g., tumor environment) that corresponds to at least the EC₅₀ (e.g., an EC₅₀ between 0.01 and 0.5 µg/ml), optionally the EC₇₀ or optionally the EC₁₀₀, for neutralization of the enzymatic activity of CD39 (e.g., an EC₁₀₀ between 0.05 and 1 µg/ml, between 0.1 and 1 µg/ml) . The antibody can for example be administered in an amount to achieve and/or maintained a concentration in circulation or in an extravascular tissue (e.g., tumor environment) of at least about 0.1 µg/ml, 0.5 µg/ml, 1 µg/ml or 2 µg/ml). For example, to achieve a concentration in an extravascular tissue of between 0.05 and 1 µg/ml, or between 0.1 and 1 µg/ml, the anti-CD39 antibody is administered in amounts effective to achieve a concentration in circulation of the anti-CD39 antibody of between 0.5 and 10 µg/ml, or between 1 and 10 µg/ml. Optionally, the anti-CD39 antibody is administered at least twice and in amounts effective to maintain the concentration of the anti-CD39 antibody at least the aforementioned concentration for at least 1 week, 2 weeks, 3 weeks, 4 weeks, between two successive administrations of the anti-CD39 antibody and/or throughout the administration cycle.

The disclosure also provides a method for treatment of disease in an individual, the treatment comprising administering to the individual an anti-CD39 antibody that neutralizes the enzymatic activity of CD39 for at least one administration cycle in which the anti-CD39 antibody is administered at least once, optionally at least twice, in an amount effective to achieve, and/or to maintain between two successive administrations of the anti-CD39 antibody, a blood or tissue concentration of anti-CD39 antibody of at least 1 µg/ml, optionally at least 10 µg/ml, optionally between 1 and 100 µg/ml. Optionally, the anti-CD39 antibody is administered at least twice and in amounts effective to maintain a continuous blood or tissue concentration of the anti-CD39 antibody of at least 1 µg/ml, optionally at least 10 µg/ml, optionally between 1 and 100 µg/ml, for at least 1 week, 2 weeks, 3 weeks, 4 weeks, between two successive administrations of the anti-CD39 antibody and/or throughout the administration cycle.

These aspects are more fully described in, and additional aspects, features, and advantages will be apparent from, the description provided herein.

### DETAILED DESCRIPTION OF THE INVENTION

### Definitions

As used in the specification, "a" or "an" may mean one or more. As used in the claim(s), when used in conjunction with the word "comprising", the words "a" or "an" may mean one or more than one. As used herein "another" may mean at least a second or more.

Where "comprising" is used, this can optionally be replaced by "consisting essentially of" or by "consisting of".

Human CD39, also known as "vascular" CD39, NTPdase1, ENTPD1, ATPDase and vascular ATP diphosphohydrolase, EC 3.6.1.5, exhibits ATPase activity. CD39 hydrolyzes extracellular ATP and ADP to AMP, which is further converted to adenosine by another enzyme, 5-prime nucleotidase. The amino acid sequence of the "vascular" human CD39 mature polypeptide chain is shown in Genbank under accession number P49961, the entire disclosure of which is incorporated herein by reference, and as follows:

1 MEDTKESNVK TFCSKNILAI LGFSSIIAVI ALLAVGLTQN KALPENVKYG IVLDAGSSHT
61 SLYIYKWPAE KENDTGVVHQ VEECRVKGPG ISKFVQKVNE IGIYLTDCME RAREVIPRSQ
121 HQETPVYLGA TAGMRLLRME SEELADRVLD VVERSLSNYP FDFQGARIIT GQEEGAYGWI
181 TINYLLGKFS QKTRWFSIVP YETNNQETFG ALDLGGASTQ VTFVPQNQTI ESPDNALQFR
241 LYGKDYNVYT HSFLCYGKDQ ALWQKLAKDI QVASNEILRD PCFHPGYKKV VNVSDLYKTP
301 CTKRFEMTLP FQQFEIQGIG NYQQCHQSIL ELFNTSYCPY SQCAFNGIFL PPLQGDFGAF
361 SAFYFVMKFL NLTSEKVSQE KVTEMMKKFC AQPWEEIKTS YAGVKEKYLS EYCFSGTYIL
421 SLLLQGYHFT ADSWEHIHFI GKIQGSDAGW TLGYMLNLTN MIPAEQPLST PLSHSTYVFL

481 MVLFSLVLFT VAIIGLLIFH KPSYFWKDMV (SEQ ID NO: 1)

In the context herein, "inhibit", "neutralize" or "neutralizing" when referring to the CD39 polypeptide (e.g., "neutralize CD39", "neutralize the activity of CD39" or "neutralize the enzymatic activity of CD39", etc.), refers to a process in which the ATP hydrolysis (ATPase) activity of CD39 is inhibited. This comprises, notably the inhibition of CD39-mediated generation of AMP and/or ADP, i.e., the inhibition of CD39-mediated catabolism of ATP to AMP and/or ADP. This can be measured for example in a cellular assay that measures the capacity of a test compound to inhibit the conversion of ATP to AMP and/or ADP, either directly or indirectly. For example, disappearance of ATP and/or generation of AMP can be assessed, as described herein. In one embodiment, an antibody preparation causes at least a 60% decrease in the conversion of ATP to AMP, at least a 70% decrease in the conversion of ATP to AMP, or at least an 80% or 90% decrease in the conversion of ATP to AMP, referring, for example, to the assays described herein (e.g., disappearance of ATP and/or generation of AMP).

Whenever within this whole specification "treatment of cancer" or the like is mentioned with reference to anti-CD39 binding agent (e.g., antibody), there is meant: (a) method of treatment of cancer, said method comprising the step of administering (for at least one treatment) an anti-CD39 binding agent, (preferably in a pharmaceutically acceptable carrier material) to an individual, a mammal, especially a human, in need of such treatment, in a dose that allows for the treatment of cancer, (a therapeutically effective amount), preferably in a dose (amount) as specified herein; (b) the use of an anti-CD39 binding agent for the treatment of cancer, or an anti-CD39 binding agent, for use in said treatment (especially in a human); (c) the use of an anti-CD39 binding agent for the manufacture of a pharmaceutical preparation for the treatment of cancer, a method of using an anti-CD39 binding agent for the manufacture of a pharmaceutical preparation for the treatment of cancer, optionally comprising admixing an anti-CD39 binding agent with a pharmaceutically acceptable carrier, or a pharmaceutical preparation comprising an effective dose of an anti-CD39 binding agent that is appropriate for the treatment of cancer; or (d) any combination of a), b), and c), in accordance with the subject matter allowable for patenting in a country where this application is filed.

The term "antibody," as used herein, refers to polyclonal and monoclonal antibodies. Depending on the type of constant domain in the heavy chains, antibodies are assigned to one of five major classes: IgA, IgD, IgE, IgG, and IgM. Several of these are further divided into subclasses or isotypes, such as IgG1, IgG2, IgG3, IgG4, and the like. An exemplary immunoglobulin (antibody) structural unit comprises a tetramer. Each tetramer is composed of two identical pairs of polypeptide chains, each pair having one "light" (about 25 kDa) and one "heavy" chain (about 50-70 kDa). The N-terminus of each chain defines a variable region of about 100 to 110 or more amino acids that is primarily responsible for antigen recognition. The terms variable light chain (V_{L}) and variable heavy chain (V_{H}) refer to these light and heavy chains respectively. The heavy-chain constant domains that correspond to the different classes of immunoglobulins are termed "alpha," "delta," "epsilon," "gamma" and "mu," respectively. The subunit structures and three-dimensional configurations of different classes of immunoglobulins are well known. IgG are the exemplary classes of antibodies employed herein because they are the most common antibodies in the physiological situation and because they are most easily made in a laboratory setting. Optionally the antibody is a monoclonal antibody. Particular examples of antibodies are humanized, chimeric, human, or otherwise-human-suitable antibodies. "Antibodies" also includes any fragment or derivative of any of the herein described antibodies.

The term "specifically binds to" means that an antibody can bind preferably in a competitive binding assay to the binding partner, e.g., CD39, as assessed using either recombinant forms of the proteins, epitopes therein, or native proteins present on the surface of isolated target cells. Competitive binding assays and other methods for determining specific binding are further described below and are well known in the art.

When an antibody is said to "compete with" a particular monoclonal antibody, it means that the antibody competes with the monoclonal antibody in a binding assay using either recombinant CD39 molecules or surface expressed CD39 molecules. For example, if a test antibody reduces the binding of a reference antibody to a CD39 polypeptide or CD39-expressing cell in a binding assay, the antibody is said to "compete" respectively with the reference antibody.

The term "affinity", as used herein, means the strength of the binding of an antibody to an epitope. The affinity of an antibody is given by the dissociation constant Kd, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Kₐ is defined by 1/Kd. Methods for determining the affinity of mAbs can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983), which references are entirely incorporated herein by reference. One standard method well known in the art for determining the affinity of mAbs is the use of surface plasmon resonance (SPR) screening (such as by analysis with a BIAcore™ SPR analytical device).

Within the context herein a "determinant" designates a site of interaction or binding on a polypeptide.

The term "epitope" refers to an antigenic determinant, and is the area or region on an antigen to which an antibody binds. A protein epitope may comprise amino acid residues directly involved in the binding as well as amino acid residues which are effectively blocked by the specific antigen binding antibody or peptide, *i.e.,* amino acid residues within the "footprint" of the antibody. It is the simplest form or smallest structural area on a complex antigen molecule that can combine with e.g., an antibody or a receptor. Epitopes can be linear or conformational/structural. The term "linear epitope" is defined as an epitope composed of amino acid residues that are contiguous on the linear sequence of amino acids (primary structure). The term "conformational or structural epitope" is defined as an epitope composed of amino acid residues that are not all contiguous and thus represent separated parts of the linear sequence of amino acids that are brought into proximity to one another by folding of the molecule (secondary, tertiary and/or quaternary structures). A conformational epitope is dependent on the 3-dimensional structure. The term 'conformational' is therefore often used interchangeably with 'structural'.

The term "deplete" or "depleting", with respect to CD39-expressing cells, means a process, method, or compound that results in killing, elimination, lysis or induction of such killing, elimination or lysis, so as to negatively affect the number of such CD39-expressing cells present in a sample or in a subject.

The term "internalization", used interchangeably with "intracellular internalization", refers to the molecular, biochemical and cellular events associated with the process of translocating a molecule from the extracellular surface of a cell to the intracellular surface of a cell. The processes responsible for intracellular internalization of molecules are well-known and can involve, *inter alia,* the internalization of extracellular molecules (such as hormones, antibodies, and small organic molecules); membrane-associated molecules (such as cell-surface receptors); and complexes of membrane-associated molecules bound to extracellular molecules (for example, a ligand bound to a transmembrane receptor or an antibody bound to a membrane-associated molecule). Thus, "inducing and/or increasing internalization" comprises events wherein intracellular internalization is initiated and/or the rate and/or extent of intracellular internalization is increased.

The term "agent" is used herein to denote a chemical compound, a mixture of chemical compounds, a biological macromolecule, or an extract made from biological materials. The term "therapeutic agent" refers to an agent that has biological activity.

For the purposes herein, a "humanized" or "human" antibody refers to an antibody in which the constant and variable framework region of one or more human immunoglobulins is fused with the binding region, e.g., the CDR, of an animal immunoglobulin. Such antibodies are designed to maintain the binding specificity of the non-human antibody from which the binding regions are derived, but to avoid an immune reaction against the non-human antibody. Such antibodies can be obtained from transgenic mice or other animals that have been "engineered" to produce specific human antibodies in response to antigenic challenge (see, e.g., Green et al. (1994) Nature Genet 7:13; Lonberg et al. (1994) Nature 368:856; Taylor et al. (1994) Int Immun 6:579, the entire teachings of which are herein incorporated by reference). A fully human antibody also can be constructed by genetic or chromosomal transfection methods, as well as phage display technology, all of which are known in the art (see, e.g., McCafferty et al. (1990) Nature 348:552-553). Human antibodies may also be generated by in vitro activated B cells (see, e.g., U.S. Pat. Nos. 5,567,610 and 5,229,275, which are incorporated in their entirety by reference).

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity.

As used herein, the term "antigen binding domain" refers to a domain comprising a three-dimensional structure capable of immunospecifically binding to an epitope. Thus, in one embodiment, said domain can comprise a hypervariable region, optionally a VH and/or VL domain of an antibody chain, optionally at least a VH domain. In another embodiment, the binding domain may comprise at least one complementarity determining region (CDR) of an antibody chain. In another embodiment, the binding domain may comprise a polypeptide domain from a non-immunoglobulin scaffold.

The term "hypervariable region" when used herein refers to the amino acid residues of an antibody that are responsible for antigen binding. The hypervariable region generally comprises amino acid residues from a "complementarity-determining region" or "CDR" (e.g., residues 24-34 (L1), 50-56 (L2) and 89-97 (L3) in the light-chain variable domain and 31-35 (H1), 50-65 (H2) and 95-102 (H3) in the heavy-chain variable domain; disclosure (see Kabat et al. (1991) Sequences of Protein of Immunological Interest, 5th ed., United States Public Health Service, National Institute of Health, Bethesda, MD)) and/or those residues from a "hypervariable loop" (e.g., residues 26-32 (L1), 50-52 (L2) and 91-96 (L3) in the light-chain variable domain and 26-32 (H1), 53-55 (H2) and 96-101 (H3) in the heavy-chain variable domain; Chothia and Lesk, J. Mol. Biol 1987;196:901-917), or a similar system for determining essential amino acids responsible for antigen binding. Using the Kabat numbering system, the actual linear amino acid sequence of a peptide may contain fewer or additional amino acids corresponding to a shortening of, or insertion into, a FR or CDR of the variable domain. For example, a heavy chain variable domain may include a single amino acid insert (residue 52a according to Kabat) after residue 52 of CDR H2 and inserted residues (e.g., residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence. Another suitable numbering system is the Abnum system. Unless otherwise specified, the Abnum amino acid numbering nomenclature for immunoglobulins is used to refer to positions in the VH and VL domains (see Abhinandan and Martin, (2008) Molecular Immunology 45: 3832-3839, the disclosure of which is incorporated by reference). Sequence numbering using the Abnum system can also be automatically generated at http://www.bioinfo.org.uk/abs/abnum. However it will be appreciated that the person of skill in the art can use an alternative numbering system and identify positions corresponding to Abnum numbering. Phrases such as "Abm position", "Abm numbering" and "according to Abm" herein refer to this numbering system for heavy chain variable domains or light chain variable domains.

By "framework" or "FR" residues as used herein is meant the region of an antibody variable domain exclusive of those regions defined as CDRs. Each antibody variable domain framework can be further subdivided into the contiguous regions separated by the CDRs (FR1, FR2, FR3 and FR4).

The terms "Fc domain," "Fc portion," and "Fc region" refer to a C-terminal fragment of an antibody heavy chain, e.g., from about amino acid (aa) 230 to about aa 450 (Kabat numbering) of human γ (gamma) heavy chain or its counterpart sequence in other types of antibody heavy chains (e.g., α, δ, ε and µ for human antibodies), or a naturally occurring allotype thereof.

The terms "isolated", "purified" or "biologically pure" refer to material that is substantially or essentially free from components which normally accompany it as found in its native state. Purity and homogeneity are typically determined using analytical chemistry techniques such as polyacrylamide gel electrophoresis or high performance liquid chromatography. A protein that is the predominant species present in a preparation is substantially purified.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

Within the context herein, the term antibody that "binds" a polypeptide or epitope designates an antibody that binds said determinant with specificity and/or affinity.

The term "identity" or "identical", when used in a relationship between the sequences of two or more polypeptides, refers to the degree of sequence relatedness between polypeptides, as determined by the number of matches between strings of two or more amino acid residues. "Identity" measures the percent of identical matches between the smaller of two or more sequences with gap alignments (if any) addressed by a particular mathematical model or computer program (i.e., "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988).

Methods for determining identity are designed to give the largest match between the sequences tested. Methods of determining identity are described in publicly available computer programs. Computer program methods for determining identity between two sequences include the GCG program package, including GAP (Devereux et al., Nucl. Acid. Res. 12, 387 (1984); Genetics Computer Group, University of Wisconsin, Madison, Wis.), BLASTP, BLASTN, and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

### Production of antibodies

The present invention is based, in part, on the discovery of modified human acceptor framework sequences into which antibody CDRs can be incorporated such that the resulting anti-CD39 variable region has high physicochemical stability and high binding affinity and potency in neutralization of the enzymatic activity of human CD39.

Anti-CD39 antibody VH and VL sequences are provided below in Table 1:

**Table 1**

| V domain | SEQ ID | Amino acid sequence |
|---|---|---|
| mAb1 VH | 6 | QVQLVQSGSELKKPGASVKVSCKASGYTFTHYGMNWVRQAPGQGLEWMGWINTYTGEPT YADDFKGRFVFSLDTSVSTAYLQISSLKAEDTAVYYCARRRYEGNYVFYYFDYWGQGTT VTVSS |
| mAb1 VL | 7 | DIQMTQSPSSLSASVGDRVTITCRASENIYSYFSWYQQKPGKAPKLLIYTAKTLAEGVP SRFSGSGSGTDFTLTISSLQPEDFATYYCQHHYVTPYTFGGGTKVEIK |
| mAb2 VH | 8 | QVQLVQSGSELKKPGASVKVSCKASGYTFTHYGMNWVRQAPGQGLKWMGWINTYTGEPT YADDFKGRFVFSLDTSVSTAYLQISSLKAEDTAVYYCARRRYEGNYVFYYFDYWGQGTT VTVSS |
| mAb2 VL | 9 | DIQMTQSPSSLSASVGDRVTITCRASENIYSYFSWYQQKPGKAPKLLIYTAKTLAEGVP SRFSGSGSGTDFTLTISSLQPEDFATYYCQHHYVTPYTFGGGTKVEIK |
| mAb3 VH | 10 | QVQLVQSGSELKKPGASVKVSCKASGYTFTHYGMNWVRQAPGKGLKWMGWINTYTGEPT YADDFKGRFVFSLDTSVSTAYLQISSLKAEDTAVYYCARRRYEGNYVFYYFDYWGQGTT VTVSS |
| mAb3 VL | 11 | DIQMTQSPSSLSASVGDRVTITCRASENIYSYFSWYQQKPGKAPKLLIYTAKTLAEGVP SRFSGSGSGTDFTLTISSLQPEDFATYYCQHHYVTPYTFGGGTKVEIK |
| mAb4 VH | 12 | QIQLVQSGSELKKPGASVKVSCKASGYTFTHYGMNWVRQAPGKGLKWMGWINTYTGEPT YADDFKGRFVFSLDASVSTAYLQISSLKAEDTAVYYCARRRYEGNYVFYYFDYWGQGTT VTVSS |
| mAb4 VL | 13 | DIQMTQSPSSLSASVGDRVTITCRASENIYSYFSWYQQKPGKAPKLLIYTAKTLAEGVP SRFSGSGSGTDFTLTISSLQPEDFATYYCQHHYVTPYTFGGGTKVEIK |
| mAb5 VH | 14 | QVQLVQSGSELKKPGASVKVSCKASGYTFTHYGMNWVRQAPGQGLKWMGWINTYTGEPT YADDFKGRFVFSLDTSVSTAYLQISSLKAEDTAVYYCARRRYEGNYVFYYFDYWGQGTT VTVSS |
| mAb5 VL | 15 | DIQMTQSPSSLSASVGDRVTITCRASENIYSYFSWYQQKPGKAPKLLVYTAKTLAEGVP SRFSGSGSGTDFTLTISSLQPEDFATYYCQHHYVTPYTFGGGTKVEIK |
| mAb6 VH | 16 | QVQLVQSGSELKKPGASVKVSCKASGYTFTHYGMNWVRQAPGKGLKWMGWINTYTGEPT YADDFKGRFVFSLDTSVSTAYLQISSLKAEDTAVYYCARRRYEGNYVFYYFDYWGQGTT VTVSS |
| mAb6 VL | 17 | DIQMTQSPSSLSASVGDRVTITCRASENIYSYFSWYQQKPGKAPKLLVYTAKTLAEGVP SRFSGSGSGTDFTLTISSLQPEDFATYYCQHHYVTPYTFGGGTKVEIK |
| mAb7 VH | 18 | QIQLVQSGSELKKPGASVKVSCKASGYTFTHYGMNWVRQAPGKGLKWMGWINTYTGEPT YADDFKGRFVFSLDASVSTAYLQISSLKAEDTAVYYCARRRYEGNYVFYYFDYWGQGTT VTVSS |
| mAb7 VL | 19 | DIQMTQSPSSLSASVGDRVTITCRASENIYSYFSWYQQKPGKAPKLLVYTAKTLAEGVP SRFSGSGSGTDFTLTISSLQPEDFATYYCQHHYVTPYTFGGGTKVEIK |

Positions in the VH and VL domains herein are described using the Abnum amino acid numbering nomenclature for immunoglobulins (see Abhinandan and Martin, (2008) Molecular Immunology 45: 3832-3839, the disclosure of which is incorporated by reference). Sequence numbering using the Abnum system can also be automatically generated at http://www.bioinfo.org.uk/abs/abnum. However it will be appreciated that the person of skill in the art can use an alternative numbering system and identify positions corresponding to Abnum numbering. For example, for Abnum residues 46 and 62 in the heavy chains, the Abnum position corresponds to the same positions in the Kabat numbering system (Kabat et al. (1991) Sequences of Protein of Immunological Interest, 5th ed., United States Public Health Service, National Institute of Health, Bethesda, MD). Abnum positions 38, 43, 46 and 62 correspond, respectively to positions 38, 43, 46 and 63 in SEQ ID NOS: 6 and 8.

In one embodiment, the antibody comprises a heavy chain framework from the human subgroup IGHV7-4-1 together with JH6, optionally the IGHV7-4-1 is IGHV7-4-1*01. In one embodiment, the humanized antibody comprises a light chain framework from the human subgroup IGKJ4 or IGKJ2, optionally from IGKJ4*01.

The antibody may further comprise one or more mutations in the human framework sequences, to, e.g., enhance affinity, stability, or other properties of the antibody.

Examples of VH and VL amino acid sequences of an anti-CD39 antibody are shown in SEQ ID NOS: 6-19, respectively. In one aspect, provided is an isolated antibody that binds a human CD39 polypeptide, wherein the antibody comprises: a HCDR1 region comprising an amino acid sequence HYGMN as set forth in SEQ ID NO: 20; a HCDR2 region comprising an amino acid sequence WINTYTGEPTYADDFKG as set forth in SEQ ID NO: 21; a HCDR3 region comprising an amino acid sequence RRYEGNYVFYYFDY as set forth in SEQ ID NO: 22; a LCDR1 region comprising an amino acid sequence RASENIYSYFS as set forth in SEQ ID NO: 23; a LCDR2 region comprising an amino acid sequence TAKTLAE as set forth in SEQ ID NO: 24; a LCDR3 region comprising an amino acid sequence QHHYVTPYT as set forth in SEQ ID NO: 25.

In one aspect, the invention provides an antigen binding domain or antibody that binds a human CD39 polypeptide, comprising:
(a) a CDR-H1 comprising the amino acid sequence of SEQ ID NO:20;
(b) a CDR-H2 comprising the amino acid sequence of SEQ ID NO:21;
(c) a CDR-H3 comprising the amino acid sequence of SEQ ID NO:22;
(d) a CDR-L1 comprising the amino acid sequence of SEQ ID NO:23;
(e) a CDR-L2 comprising the amino acid sequence of SEQ ID NO:24;
(f) a CDR-L3 comprising the amino acid sequence of SEQ ID NO:25; and
(g) human heavy and light chain framework sequences.

In one embodiment, the antibody comprises a heavy chain framework from the human subgroup IGHV7-4-1 together with IGHJ6, optionally the antibodies comprises IGHV7-4-1, together with IGHJ6. In one embodiment, the humanized antibody comprises a light chain framework from the human subgroup IGKJ2 or IGKJ4, optionally IGKJ4*01.

In one aspect of any anti-CD39 antibody embodiment herein, the heavy chain variable region comprises a lysine residue at position 46 (Abm numbering), e.g., the variable region comprises a E46K substitution and an aspartic acid at Abm residue 62 (CDR2).

Optionally a human framework comprises one or more mutations, e.g., back mutations to introduce a residue present at the particular position in a non-human mammal (e.g., a mouse or a rat).

In one aspect, provided is an isolated humanized antibody that binds human CD39 polypeptide, comprising:
(a) a CDR-H1 comprising the amino acid sequence of SEQ ID NO: 20;
(b) a CDR-H2 comprising the amino acid sequence of SEQ ID NO: 21;
(c) a CDR-H3 comprising the amino acid sequence of SEQ ID NO: 22;
(d) a CDR-L1 comprising the amino acid sequence of SEQ ID NO: 23;
(e) a CDR-L2 comprising the amino acid sequence of SEQ ID NO: 24;
(f) a CDR-L3 comprising the amino acid sequence of SEQ ID NO: 25; and
(g) human framework sequences, wherein a lysine (K) residue is present at position 46 of the VH domain.

In one aspect, provided is an isolated humanized antibody that binds human CD39 polypeptide, comprising:
(a) a CDR-H1 comprising the amino acid sequence of SEQ ID NO: 20;
(b) a CDR-H2 comprising the amino acid sequence of SEQ ID NO: 21;
(c) a CDR-H3 comprising the amino acid sequence of SEQ ID NO: 22;
(d) a CDR-L1 comprising the amino acid sequence of SEQ ID NO: 23;
(e) a CDR-L2 comprising the amino acid sequence of SEQ ID NO: 24;
(f) a CDR-L3 comprising the amino acid sequence of SEQ ID NO: 25; and
(g) human framework sequences, wherein an arginine (R) residues is present as position 38 of the VH domain, a glutamine (Q) residue is present at position 43 of the VH domain, and a glutamic acid (E) residue is present at position 46 of the VH domain.

In any embodiment, the antibody comprises a heavy chain framework from the human subgroup IGHV7-4-1 together with IGHJ6, optionally the antibodies comprises IGHV7-4-1, together with IGHJ6. In one embodiment, the humanized antibody comprises a light chain framework from the human subgroup IGKJ2 or IGKJ4, optionally IGKJ4*01.

The lysine (K) residue at position 46 may exist naturally in the human VH framework sequence, or may be introduced by amino acid substitution or other modification of the sequence.

In another aspect, the anti-CD39 antibody comprises a VH domain having at least about 80% sequence identity (e.g., at least about 85%, 90%, 95%, 97%, 98%, or more identity) to the VH domain of SEQ ID NOS: 6, 8 or 12. In another aspect, the anti-CD39 antibody comprises a VL domain having at least about 80% sequence identity (e.g., at least about 85%, 90%, 95%, 97%, 98%, or more identity) to the VH domain of SEQ ID NOS: 7 or 15.

DNA encoding an antibody can be prepared and placed in an appropriate expression vector for transfection into an appropriate host. The host is then used for the recombinant production of the antibody, or variants thereof, such as a humanized version of that monoclonal antibody, active fragments of the antibody, chimeric antibodies comprising the antigen recognition portion of the antibody, or versions comprising a detectable moiety.

DNA encoding the monoclonal antibodies of the disclosure can be readily isolated and sequenced using conventional procedures (e. g., by using oligonucleotide probes that are capable of binding specifically to genes encoding the heavy and light chains of murine antibodies). In one aspect, provided is a nucleic acid encoding a heavy chain or a light chain of an anti-CD39 antibody of any embodiment herein. Once isolated, the DNA can be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. As described elsewhere in the present specification, such DNA sequences can be modified for any of a large number of purposes, e.g., for humanizing antibodies, producing fragments or derivatives, or for modifying the sequence of the antibody, e.g., in the antigen binding site in order to optimize the binding specificity of the antibody. In one embodiment, provided is an isolated nucleic acid sequence encoding a light chain and/or a heavy chain of an antibody, as well as a recombinant host cell comprising (e.g., in its genome) such nucleic acid. Recombinant expression in bacteria of DNA encoding the antibody is well known in the art (see, for example, Skerra et al., Curr. Opinion in Immunol., 5, pp. 256 (1993); and Pluckthun, Immunol. 130, p. 151 (1992).

Once antibodies are identified that are capable of binding CD39 and/or having other desired properties, they will also typically be assessed, using methods such as those described herein, for their ability to bind to other polypeptides, including unrelated polypeptides. Ideally, the antibodies bind with substantial affinity only to CD39, and do not bind at a significant level to unrelated polypeptides, or other polypeptides of the NTPDase family, notably CD39-L1, L2, L3 and L4 or NTPDase8. However, it will be appreciated that, as long as the affinity for CD39 is substantially greater (e.g., 10x, 100x, 500x, 1000x, 10,000x, or more) than it is for other, unrelated polypeptides), then the antibodies are suitable for use in the present methods.

Typically, an anti-CD39 antibody provided herein has an affinity for a CD39 polypeptide (e.g., a monomeric CD39 polypeptide as produced in the Examples herein) in the range of about 10⁴ to about 10¹¹ M⁻¹ (e.g., about 10⁸ to about 10¹⁰ M⁻¹). For example, in a particular aspect the disclosure provides Anti-CD39 antibody that have an average disassociation constant (K_{D}) of less than 1 x 10⁻⁹ M with respect to CD39, as determined by, e.g., surface plasmon resonance (SPR) screening (such as by analysis with a BIAcore™ SPR analytical device). In a more particular exemplary aspect, the disclosure provides anti-CD39 antibodies that have a KD of about 1 x 10⁻⁸ M to about 1 x 10⁻¹⁰ M, or about 1 x 10⁻⁹ M to about 1 x 10⁻¹¹ M, for CD39.

Antibodies can be characterized for example by a mean KD of no more than about (i.e. better affinity than) 100, 60, 10, 5, or 1 nanomolar, preferably sub-nanomolar or optionally no more than about 500, 200, 100 or 10 picomolar. KD can be determined for example by surface plasmon resonance, for example by immobilizing recombinantly produced human CD39 proteins on a chip surface, followed by application of the antibody to be tested in solution. In one embodiment, the method further comprises a step (d), selecting antibodies from (b) that are capable of competing for binding to CD39 with control antibody.

Where the test antibodies have modifications in their VH and/VL from specified VH and VL amino acid sequences, a simple competition assay may be employed in which the control (the antibody having a VH and VL of SEQ ID NOS: 6 and 7, or the antibody having a VH and VL of SEQ ID NOS: 8 and 9, for example) and test antibodies are admixed (or pre-adsorbed) and applied to a sample containing CD39 polypeptides. Protocols based upon western blotting and the use of BIACORE™ analysis are suitable for use in such competition studies.

In certain embodiments, one pre-mixes the control antibodies with varying amounts of the test antibodies (e.g., about 1:10 or about 1:100) for a period of time prior to applying to the CD39 antigen sample. In other embodiments, the control and varying amounts of test antibodies can simply be admixed during exposure to the CD39 antigen sample. As long as one can distinguish bound from free antibodies (e. g., by using separation or washing techniques to eliminate unbound antibodies) and control antibody from the test antibodies (e. g., by using species-specific or isotype-specific secondary antibodies or by specifically labelling control antibody with a detectable label) one can determine if the test antibodies reduce the binding of control antibody to the antigens, indicating that the test antibody ay recognize substantially the same epitope as control antibody. The binding of the (labelled) control antibodies in the absence of a completely irrelevant antibody can serve as the control high value. The control low value can be obtained by incubating the labelled control antibodies with unlabelled antibodies of exactly the same type, where competition would occur and reduce binding of the labelled antibodies. In a test assay, a significant reduction in labelled antibody reactivity in the presence of a test antibody is indicative of a test antibody that "cross-reacts" or competes with the labelled control antibody. A test antibody may reduce the binding of control antibody to CD39 antigens by at least about 50%, such as at least about 60%, or more preferably at least about 80% or 90% (e. g., about 65-100%), at any ratio of control antibody:test antibody between about 1:10 and about 1:100. Preferably, such test antibody will reduce the binding of control antibody to the CD39 antigen by at least about 90% (e.g., about 95%).

Competition can also be assessed by, for example, a flow cytometry test. In such a test, cells bearing a given CD39 polypeptide can be incubated first with control antibody, for example, and then with the test antibody labelled with a fluorochrome or biotin. The antibody is said to compete with control antibody if the binding obtained upon preincubation with a saturating amount of control antibody is about 80%, preferably about 50%, about 40% or less (e.g., about 30%, 20% or 10%) of the binding (as measured by mean of fluorescence) obtained by the antibody without preincubation with control antibody. Alternatively, an antibody is said to compete with control antibody if the binding obtained with a labelled control antibody antibody (by a fluorochrome or biotin) on cells preincubated with a saturating amount of test antibody is about 80%, preferably about 50%, about 40%, or less (e.g., about 30%, 20% or 10%) of the binding obtained without preincubation with the test antibody.

A simple competition assay in which a test antibody is pre-adsorbed and applied at saturating concentration to a surface onto which a CD39 antigen is immobilized may also be employed. The surface in the simple competition assay can be a BIACORE™ chip (or other media suitable for surface plasmon resonance analysis). The control antibody (the antibody having a VH and VL of SEQ ID NOS: 6 and 7, or the antibody having a VH and VL of SEQ ID NOS: 8 and 9, for example) is then brought into contact with the surface at a CD39-saturating concentration and the CD39 and surface binding of the control antibody is measured. This binding of the control antibody is compared with the binding of the control antibody to the CD39-containing surface in the absence of test antibody. In a test assay, a significant reduction in binding of the CD39-containing surface by the control antibody in the presence of a test antibody indicates that the test antibody may recognize substantially the same epitope as the control antibody such that the test antibody "cross-reacts" with the control antibody. A test antibody may reduce the binding of control antibody to a CD39 antigen by at least about 30% or more, preferably about 40%. Preferably, such a test antibody will reduce the binding of the control antibody to the CD39 antigen by at least about 50% (e. g., at least about 60%, at least about 70%, or more). It will be appreciated that the order of control and test antibodies can be reversed: that is, the control antibody can be first bound to the surface and the test antibody is brought into contact with the surface thereafter in a competition assay. Preferably, the antibody having higher affinity for the CD39 antigen is bound to the surface first, as it will be expected that the decrease in binding seen for the second antibody (assuming the antibodies are cross-reacting) will be of greater magnitude. Further examples of such assays are provided in, e.g., Saunal (1995) J. Immunol. Methods 183: 33-41, the disclosure of which is incorporated herein by reference.

Determination of whether an antibody binds within an epitope region can be carried out in ways known to the person skilled in the art. As one example of such mapping/characterization methods, an epitope region for an anti-CD39 antibody may be determined by epitope "foot-printing" using chemical modification of the exposed amines/carboxyls in the CD39 protein. One specific example of such a foot-printing technique is the use of HXMS (hydrogen-deuterium exchange detected by mass spectrometry) wherein a hydrogen/deuterium exchange of receptor and ligand protein amide protons, binding, and back exchange occurs, wherein the backbone amide groups participating in protein binding are protected from back exchange and therefore will remain deuterated. Relevant regions can be identified at this point by peptic proteolysis, fast microbore high-performance liquid chromatography separation, and/or electrospray ionization mass spectrometry. See, e. g., Ehring H, Analytical Biochemistry, Vol. 267 (2) pp. 252-259 (1999) Engen, J. R. and Smith, D. L. (2001) Anal. Chem. 73, 256A-265A. Another example of a suitable epitope identification technique is nuclear magnetic resonance epitope mapping (NMR), where typically the position of the signals in two-dimensional NMR spectra of the free antigen and the antigen complexed with the antigen binding peptide, such as an antibody, are compared. The antigen typically is selectively isotopically labeled with 15N so that only signals corresponding to the antigen and no signals from the antigen binding peptide are seen in the NMR-spectrum. Antigen signals originating from amino acids involved in the interaction with the antigen binding peptide typically will shift position in the spectrum of the complex compared to the spectrum of the free antigen, and the amino acids involved in the binding can be identified that way. See, e. g., Ernst Schering Res Found Workshop. 2004; (44): 149-67; Huang et al., Journal of Molecular Biology, Vol. 281 (1) pp. 61-67 (1998); and Saito and Patterson, Methods. 1996 Jun; 9 (3): 516-24.

Epitope mapping/characterization also can be performed using mass spectrometry methods. See, e.g., Downard, J Mass Spectrom. 2000 Apr; 35 (4): 493-503 and Kiselar and Downard, Anal Chem. 1999 May 1; 71 (9): 1792-1801. Protease digestion techniques also can be useful in the context of epitope mapping and identification. Antigenic determinant-relevant regions/sequences can be determined by protease digestion, e.g., by using trypsin in a ratio of about 1:50 to CD39 or o/n digestion at and pH 7-8, followed by mass spectrometry (MS) analysis for peptide identification. The peptides protected from trypsin cleavage by the anti-CD39 binder can subsequently be identified by comparison of samples subjected to trypsin digestion and samples incubated with antibody and then subjected to digestion by e.g., trypsin (thereby revealing a footprint for the binder). Other enzymes like chymotrypsin, pepsin, etc., also or alternatively can be used in similar epitope characterization methods. Moreover, enzymatic digestion can provide a quick method for analyzing whether a potential antigenic determinant sequence is within a region of the CD39 polypeptide that is not surface exposed and, accordingly, most likely not relevant in terms of immunogenicity/antigenicity.

Site-directed mutagenesis is another technique useful for elucidation of a binding epitope. For example, in "alanine-scanning", each residue within a protein segment is replaced with an alanine residue, and the consequences for binding affinity measured. If the mutation leads to a significant reduction in binding affinity, it is most likely involved in binding. Monoclonal antibodies specific for structural epitopes (i.e., antibodies which do not bind the unfolded protein) can be used to verify that the alanine-replacement does not influence overall fold of the protein. See, e.g., Clackson and Wells, Science 1995; 267:383-386; and Wells, Proc Natl Acad Sci USA 1996; 93:1-6.

Electron microscopy can also be used for epitope "foot-printing". For example, Wang et al., Nature 1992; 355:275-278 used coordinated application of cryoelectron micros-copy, three-dimensional image reconstruction, and X-ray crystallography to determine the physical footprint of a Fab-fragment on the capsid surface of native cowpea mosaic virus.

Other forms of "label-free" assay for epitope evaluation include surface plasmon resonance (SPR, BIACORE™) and reflectometric interference spectroscopy (RifS). See, e.g., Fägerstam et al., Journal Of Molecular Recognition 1990;3:208-14; Nice et al., J. Chroma-togr. 1993; 646:159-168; Leipert et al., Angew. Chem. Int. Ed. 1998; 37:3308-3311; Kröger et al., Biosensors and Bioelectronics 2002; 17:937-944.

It should also be noted that an antibody binding the same or substantially the same epitope as an antibody can be identified in one or more of the exemplary competition assays described herein.

In one embodiment, the anti-CD39 antibodies can be prepared such that they do not have substantial specific binding to human Fcγ receptors, e.g., any one or more of CD16A, CD16B, CD32A, CD32B and/or CD64). Such antibodies may comprise constant regions of various heavy chains that are known to lack or have low binding to Fcγ receptors. Alternatively, antibody fragments that do not comprise (or comprise portions of) constant regions, such as F(ab')2 fragments, can be used to avoid Fc receptor binding. Fc receptor binding can be assessed according to methods known in the art, including for example testing binding of an antibody to Fc receptor protein in a BIACORE™ assay. Also, generally any antibody IgG isotype can be used in which the Fc portion is modified (e.g., by introducing 1, 2, 3, 4, 5 or more amino acid substitutions) to minimize or eliminate binding to Fc receptors (see, e.g., WO 03/101485, the disclosure of which is herein incorporated by reference). Assays such as cell based assays, to assess Fc receptor binding are well known in the art, and are described in, e.g., WO 03/101485.

In one embodiment, the antibody can comprise one or more specific mutations in the Fc region that result in "Fc silent" antibodies that have minimal interaction with effector cells. Silenced effector functions can be obtained by mutation in the Fc region of the antibodies and have been described in the art: N297A mutation, the LALA mutations, (Strohl, W., 2009, Curr. Opin. Biotechnol. vol. 20(6):685-691); and D265A (Baudino et al., 2008, J. Immunol. 181 : 6664-69) see also Heusser et al., WO2012/065950, the disclosures of which are incorporated herein by reference. In one embodiment, an antibody comprises one, two, three or more amino acid substitutions in the hinge region. In one embodiment, the antibody is an IgG1 or IgG2 and comprises one, two or three substitutions at residues 233-236, optionally 233-238 (EU numbering). In one embodiment, the antibody is an IgG4 and comprises one, two or three substitutions at residues 327, 330 and/or 331 (EU numbering). Examples of silent Fc IgG1 antibodies are the LALA mutant comprising L234A and L235A mutation in the IgG1 Fc amino acid sequence. Another example of an Fc silent mutation is a mutation at residue D265, or at D265 and P329 for example as used in an IgG1 antibody as the DAPA (D265A, P329A) mutation (US 6,737,056). Another silent IgG1 antibody comprises a mutation at residue N297 (e.g. N297A, N297S mutation), which results in aglycosylated/non-glycosylated antibodies. Other silent mutations include: substitutions at residues L234 and G237 (L234A/G237A); substitutions at residues S228, L235 and R409 (S228P/L235E/R409K,T,M,L); substitutions at residues H268, V309, A330 and A331 (H268Q/V309L/A330S/A331S); substitutions at residues C220, C226, C229 and P238 (C220S/C226S/C229S/P238S); substitutions at residues C226, C229, E233, L234 and L235 (C226S/C229S/E233P/L234V/L235A; substitutions at residues K322, L235 and L235 (K322A/L234A/L235A); substitutions at residues L234, L235 and P331 (L234F/L235E/P331S); substitutions at residues 234, 235 and 297; substitutions at residues E318, K320 and K322 (L235E/E318A/K320A/K322A); substitutions at residues (V234A, G237A, P238S); substitutions at residues 243 and 264; substitutions at residues 297 and 299; substitutions such that residues 233, 234, 235, 237, and 238 defined by the EU numbering system, comprise a sequence selected from PAAAP, PAAAS and SAAAS (see WO2011/066501).

In one embodiment, the antibody can comprise one or more specific mutations in the Fc region that result in improved stability of an antibody of the disclosure, e.g. comprising multiple aromatic amino acid residues and/or having high hydrophobicity. For example, such an antibody can comprise an Fc domain of human IgG1 origin, comprises a mutation at Kabat residue(s) 234, 235, 237, 330 and/or 331. One example of such an Fc domain comprises substitutions at Kabat residues L234, L235 and P331 (e.g, L234A/L235E/P331S or (L234F/L235E/P331S). Another example of such an Fc domain comprises substitutions at Kabat residues L234, L235, G237 and P331 (e.g., L234A/L235E/G237A/P331S). Another example of such an Fc domain comprises substitutions at Kabat residues L234, L235, G237, A330 and P331 (e.g., L234A/L235E/G237A/A330S/P331S). In one embodiment, the antibody comprises an Fc domain, optionally of human IgG1 isotype, comprising: a L234X₁ substitution, a L235X₂ substitution, and a P331X₃ substitution, wherein X₁ is any amino acid residue other than leucine, X₂ is any amino acid residue other than leucine, and X₃ is any amino acid residue other than proline; optionally wherein X₁ is an alanine or phenylalanine or a conservative substitution thereof; optionally wherein X₂ is glutamic acid or a conservative substitution thereof; optionally wherein X₃ is a serine or a conservative substitution thereof. In another embodiment, the antibody comprises an Fc domain, optionally of human IgG1 isotype, comprising: a L234X₁ substitution, a L235X₂ substitution, a G237X₄ substitution and a P331X₄ substitution, wherein X₁ is any amino acid residue other than leucine, X₂ is any amino acid residue other than leucine, X₃ is any amino acid residue other than glycine, and X₄ is any amino acid residue other than proline; optionally wherein X₁ is an alanine or phenylalanine or a conservative substitution thereof; optionally wherein X₂ is glutamic acid or a conservative substitution thereof; optionally, X₃ is alanine or a conservative substitution thereof; optionally X₄ is a serine or a conservative substitution thereof. In another embodiment, the antibody comprises an Fc domain, optionally of human IgG1 isotype, comprising: a L234X₁ substitution, a L235X₂ substitution, a G237X₄ substitution, G330X₄ substitution, and a P331X₅ substitution, wherein X₁ is any amino acid residue other than leucine, X₂ is any amino acid residue other than leucine, X₃ is any amino acid residue other than glycine, X₄ is any amino acid residue other than alanine, and X₅ is any amino acid residue other than proline; optionally wherein X₁ is an alanine or phenylalanine or a conservative substitution thereof; optionally wherein X₂ is glutamic acid or a conservative substitution thereof; optionally, X₃ is alanine or a conservative substitution thereof; optionally, X₄ is serine or a conservative substitution thereof; optionally X₅ is a serine or a conservative substitution thereof. In the shorthand notation used here, the format is: Wild type residue: Position in polypeptide: Mutant residue, wherein residue positions are indicated according to EU numbering according to Kabat.

In one embodiment, an antibody comprises a heavy chain constant region comprising the amino acid sequence below, or an amino acid sequence at least 90%, 95% or 99% identical thereto but retaining the amino acid residues at Kabat positions 234, 235 and 331 (underlined):

A S T K G P S V F P L A P S S K S T S G G T A A L G C L V K D Y F P
E P V T V S W N S G A L T S G V H T F P A V L Q S S G L Y S L S S V
V T V P S S S L G T Q T Y I C N V N H K P S N T K V D K R V E P K S
C D K T H T C P P C P A P E A E G G P S V F L F P P K P K D T L M I
S R T P E V T C V V V D V S H E D P E V K F N W Y V D G V E V H N A
K T K P R E E Q Y N S T Y R V V S V L T V L H Q D W L N G K E Y K C
K V S N K A L P A S I E K T I S K A K G Q P R E P Q V Y T L P P S R
E E M T K N Q V S L T C L V K G F Y P S D I A V E W E S N G Q P E N
N Y K T T P P V L D S D G S F F L Y S K L T V D K S R W Q Q G N V F
S C S V M H E A L H N H Y T Q K S L S L S P G K (SEQ ID NO: 30)

In one embodiment, an antibody comprises a heavy chain constant region comprising the amino acid sequence below, or an amino acid sequence at least 90%, 95% or 99% identical thereto but retaining the amino acid residues at Kabat positions 234, 235 and 331 (underlined):

A S T K G P S V F P L A P S S K S T S G G T A A L G C L V K D Y F P
E P V T V S W N S G A L T S G V H T F P A V L Q S S G L Y S L S S V
V T V P S S S L G T Q T Y I C N V N H K P S N T K V D K R V E P K S
C D K T H T C P P C P A P E F E G G P S V F L F P P K P K D T L M I
S R T P E V T C V V V D V S H E D P E V K F N W Y V D G V E V H N A
K T K P R E E Q Y N S T Y R V V S V L T V L H Q D W L N G K E Y K C
K V S N K A L P A S I E K T I S K A K G Q P R E P Q V Y T L P P S R
E E M T K N Q V S L T C L V K G F Y P S D I A V E W E S N G Q P E N
N Y K T T P P V L D S D G S F F L Y S K L T V D K S R W Q Q G N V F
S C S V M H E A L H N H Y T Q K S L S L S P G K (SEQ ID NO: 31)

In one embodiment, an antibody comprises a heavy chain constant region comprising the amino acid sequence below, or an amino acid sequence at least 90%, 95% or 99% identical thereto but retaining the amino acid residues at Kabat positions 234, 235, 237, 330 and 331 (underlined):

A S T K G P S V F P L A P S S K S T S G G T A A L G C L V K D Y F P
E P V T V S W N S G A L T S G V H T F P A V L Q S S G L Y S L S S V
V T V P S S S L G T Q T Y I C N V N H K P S N T K V D K R V E P K S
C D K T H T C P P C P A P E A E G A P S V F L F P P K P K D T L M I

S R T P E V T C V V V D V S H E D P E V K F N W Y V D G V E V H N A
K T K P R E E Q Y N S T Y R V V S V L T V L H Q D W L N G K E Y K C
K V S N K A L P S S I E K T I S K A K G Q P R E P Q V Y T L P P S R
E E M T K N Q V S L T C L V K G F Y P S D I A V E W E S N G Q P E N
N Y K T T P P V L D S D G S F F L Y S K L T V D K S R W Q Q G N V F
S C S V M H E A L H N H Y T Q K S L S L S P G K (SEQ ID NO: 32)

In one embodiment, an antibody comprises a heavy chain constant region comprising the amino acid sequence below, or a sequence at least 90%, 95% or 99% identical thereto but retaining the amino acid residues at Kabat positions 234, 235, 237 and 331 (underlined):

A S T K G P S V F P L A P S S K S T S G G T A A L G C L V K D Y F P
E P V T V S W N S G A L T S G V H T F P A V L Q S S G L Y S L S S V
V T V P S S S L G T Q T Y I C N V N H K P S N T K V D K R V E P K S
C D K T H T C P P C P A P E A E G A P S V F L F P P K P K D T L M I
S R T P E V T C V V V D V S H E D P E V K F N W Y V D G V E V H N A
K T K P R E E Q Y N S T Y R V V S V L T V L H Q D W L N G K E Y K C
K V S N K A L P A S I E K T I S K A K G Q P R E P Q V Y T L P P S R
E E M T K N Q V S L T C L V K G F Y P S D I A V E W E S N G Q P E N
N Y K T T P P V L D S D G S F F L Y S K L T V D K S R W Q Q G N V F
S C S V M H E A L H N H Y T Q K S L S L S P G K (SEQ ID NO: 33)

Fc silent antibodies result in no or low ADCC activity, meaning that an Fc silent antibody exhibits an ADCC activity that is below 50% specific cell lysis. Preferably an antibody substantially lacks ADCC activity, e.g., the Fc silent antibody exhibits an ADCC activity (specific cell lysis) that is below 5% or below 1 %. Fc silent antibodies can also result in lack of FcγR-mediated cross-linking of CD39 at the surface of a CD39-expression.

In one embodiment, the antibody has a substitution in a heavy chain constant region at any one, two, three, four, five or more of residues selected from the group consisting of: 220, 226, 229, 233, 234, 235, 236, 237, 238, 243, 264, 268, 297, 298, 299, 309, 310, 318, 320, 322, 327, 330, 331 and 409 (numbering of residues in the heavy chain constant region is according to EU numbering according to Kabat). In one embodiment, the antibody comprises a substitution at residues 234, 235 and 322. In one embodiment, the antibody has a substitution at residues 234, 235 and 331. In one embodiment, the antibody has a substitution at residues 234, 235, 237 and 331. In one embodiment, the antibody has a substitution at residues 234, 235, 237, 330 and 331. In one embodiment, the Fc domain is of human IgG1 subtype. Amino acid residues are indicated according to EU numbering according to Kabat.

In one embodiment, the antibody comprises an Fc domain comprising an amino acid substitution that increases binding to human FcRn polypeptides in order to increase the in vivo half-life of the antibody. Exemplary mutations are described in Strohl, W., 2009, Curr. Opin. Biotechnol. vol. 20(6):685-691, the disclosure of which is incorporated herein by reference. Examples of substitutions used in antibodies of human IgG1 isotype are substitutions at residues M252, S254 and T256; substitutions at residues T250 and M428; substitutions at residue N434; substitutions at residues H433 and N434; substitutions at residues T307, E380 and N434; substitutions at residues T307, E380, and N434; substitutions at residues M252, S254, T256, H433, N434 and 436; substitutions at residue I253; substitutions at residues P257, N434, D376 and N434.

In one embodiment, the antibody comprises an Fc domain comprising an amino acid substitution that confers decreased sensitivity to cleavage by proteases. Matrix metalloproteinases (MMPs) represent the most prominent family of proteinases associated with tumorigenesis. While cancer cells can express MMPs, the bulk of the extracellular MMP is provided by different types of stromal cells that infiltrate the tumor and each produce a specific set of proteinases and proteinase inhibitors, which are released into the extracellular space and specifically alter the milieu around the tumor. The MMPs present in the tumor microenvironment can cleave antibodies within the hinge region and may thus lead to the inactivation of therapeutic antibodies that are designed to function within the tumor site. In one embodiment, the Fc domain comprising an amino acid substitution has decreased sensitivity to cleavage by any one, two, three or more (or all of) of the proteases selected from the group consisting of: GluV8, IdeS, gelatinase A (MMP2), gelatinase B (MMP-9), matrix metalloproteinase-7 (MMP-7), stromelysin (MMP-3), and macrophage elastase (MMP-12). In one embodiment, the antibody decreased sensitivity to cleavage comprises an Fc domain comprising an amino acid substitution at residues E233-L234 and/or L235. In one embodiment, the antibody comprises an Fc domain comprising an amino acid substitution at residues E233, L234, L235 and G236. In one embodiment, the antibody comprises an Fc domain comprising an amino acid substitution at one or more residues 233-238, e.g., such that E233-L234-L235-G236 sequence is replaced by P233-V234-A235 (G236 is deleted). See, e.g., WO99/58572 and WO2012087746, the disclosures of which are incorporated herein by reference.

An antigen-binding compound can at any desired stage be assessed for its ability to inhibit the enzymatic activity of CD39, notably to block the ATPase activity of CD39 and to reduce the production of ADP, AMP and/or adenosine by a CD39-expressing cell, and in turn restore the activity of and/or relieve the adenosine-mediated inhibition of lymphocytes.

The inhibitory activity (e.g., immune enhancing potential) of an antibody can be assessed for example, in an assay to detect the disappearance (hydrolysis) of ATP and/or the generation of AMP. In one aspect, an assay is used that is insensitive to CD39 down-modulation is used. An example of such an assay is assessing generation of AMP by detection AMP after incubating CD39-expressing cells (e.g., Ramos cells) with a test antibody, and measuring in supernatants the generation of AMP by mass spectrometry (e.g., MALDI TOF).

Other assays that are sensitive to down-modulation can also be used. For example, remaining exogenous ATP can be assessed; such an assay is directly correlated to CD39 expression. The ATPlite assay can be used, based on a luciferase reaction that is inhibited by AMP. For example, hydrolysis of ATP can be detected by bringing CD39-expressing cells into contact with a test antibody, and ATP remaining measured in supernatants using a luciferase-based assay, for example the Luminescence ATP Detection Assay System (ATPlite™, Perkin Elmer Corp.) based on firefly (Photinus pyralis) luciferase.

A decrease in hydrolysis of ATP into AMP, and/or a decrease in generation of AMP, in the presence of antibody indicate the antibody inhibits CD39. In one embodiment, an antibody preparation is capable of causing at least a 60% decrease in the enzymatic activity of a CD39 polypeptide, preferably at least a 70%, 80% or 90% decrease in the enzymatic activity of a CD39 polypeptide.

The activity of an antibody can also be measured in an indirect assay for its ability to modulate the activity of leukocytes (e.g., A2A-receptor expressing cells), for example to relieve the adenosine-mediated inhibition of lymphocyte activity, or to cause the activation of lymphocyte activity. This can be addressed, for example, using a cytokine-release assay. In another example, an antibody can be evaluated in an indirect assay for its ability to modulate the proliferation of lymphocytes.

The antibody can be tested for its ability to internalize or to induce down-modulation of CD39, e.g., whether by internalization or induction of CD39 shedding from the cell surface. Whether an anti-CD39 antibody internalizes upon binding CD39 on a mammalian cell, or whether a CD39 polypeptide undergoes intracellular internalization (e.g., upon being bound by an antibody) can be determined. In other examples, to test internalization in vivo, the test antibody is labeled and introduced into an animal known to have CD39 expressed on the surface of certain cells. The antibody can be radiolabeled or labeled with fluorescent or gold particles, for instance. Animals suitable for this assay include a mammal such as a nude mouse that contains a human CD39-expressing B cells, T cells, TReg cells, tumor transplant or xenograft, or a mouse into which cells transfected with human CD39 have been introduced, or a transgenic mouse expressing the human CD39 transgene. Appropriate controls include animals that did not receive the test antibody or that received an unrelated antibody, and animals that received an antibody to another antigen on the cells of interest, which antibody is known to be internalized upon binding to the antigen. The antibody can be administered to the animal, e.g., by intravenous injection. At suitable time intervals, tissue sections of the animal can be prepared using known methods or as described in the experimental examples below, and analyzed by light microscopy or electron microscopy, for internalization as well as the location of the internalized antibody in the cell. For internalization in vitro, the cells can be incubated in tissue culture dishes in the presence or absence of the relevant antibodies added to the culture media and processed for microscopic analysis at desired time points. The presence of an internalized, labeled antibody in the cells can be directly visualized by microscopy or by autoradiography if radiolabeled antibody is used. Optionally, in microscopy, co-localization with a known polypeptide or other cellular component can be assessed; for example co-localization with endosomal/lysosomal marker LAMP-1 (CD107a) can provide information about the subcellular localization of the internalized antibody. Alternatively, in a quantitative biochemical assay, a population of cells comprising CD39-expressing cells are contacted in vitro or in vivo with a radiolabeled test antibody and the cells (if contacted in vivo, cells are then isolated after a suitable amount of time) are treated with a protease or subjected to an acid wash to remove un-internalized antibody on the cell surface. The cells are ground up and the amount of protease resistant, radioactive counts per minute (cpm) associated with each batch of cells is measured by passing the homogenate through a scintillation counter. Based on the known specific activity of the radiolabeled antibody, the number of antibody molecules internalized per cell can be deduced from the scintillation counts of the ground- up cells. Cells are "contacted" with antibody in vitro preferably in solution form such as by adding the cells to the cell culture media in the culture dish or flask and mixing the antibody well with the media to ensure uniform exposure of the cells to the antibody.

Fragments and derivatives of antibodies (which are encompassed by the term "antibody" or "antibodies" as used in this application, unless otherwise stated or clearly contradicted by context) can be produced by techniques that are known in the art. "Fragments" comprise a portion of the intact antibody, generally the antigen binding site or variable region. Examples of antibody fragments include Fab, Fab', Fab'-SH, F (ab') 2, and Fv fragments; diabodies; any antibody fragment that is a polypeptide having a primary structure consisting of one uninterrupted sequence of contiguous amino acid residues (referred to herein as a "single-chain antibody fragment" or "single chain polypeptide").

An anti-CD39 antibody can be incorporated in a pharmaceutical formulation comprising in a concentration from 1 mg/ml to 500 mg/ml, wherein said formulation has a pH from 2.0 to 10.0. The formulation may further comprise a buffer system, preservative(s), tonicity agent(s), chelating agent(s), stabilizers and surfactants. In one embodiment, the pharmaceutical formulation is an aqueous formulation, i.e., formulation comprising water. Such formulation is typically a solution or a suspension. In a further embodiment, the pharmaceutical formulation is an aqueous solution. The term "aqueous formulation" is defined as a formulation comprising at least 50 %w/w water. Likewise, the term "aqueous solution" is defined as a solution comprising at least 50 %w/w water, and the term "aqueous suspension" is defined as a suspension comprising at least 50 %w/w water.

In another embodiment, the pharmaceutical formulation is a freeze-dried formulation, whereto the physician or the patient adds solvents and/or diluents prior to use.

In another embodiment, the pharmaceutical formulation is a dried formulation (e.g., freeze-dried or spray-dried) ready for use without any prior dissolution.

In a further aspect, the pharmaceutical formulation comprises an aqueous solution of such an antibody, and a buffer, wherein the antibody is present in a concentration from 1 mg/ml or above, and wherein said formulation has a pH from about 2.0 to about 10.0.

In a another embodiment, the pH of the formulation is in the range selected from the list consisting of from about 2.0 to about 10.0, about 3.0 to about 9.0, about 4.0 to about 8.5, about 5.0 to about 8.0, and about 5.5 to about 7.5.

In a further embodiment, the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative embodiment.

In a further embodiment, the formulation further comprises a pharmaceutically acceptable preservative. In a further embodiment, the formulation further comprises an isotonic agent. In a further embodiment, the formulation also comprises a chelating agent. In a further embodiment the formulation further comprises a stabilizer. In a further embodiment, the formulation further comprises a surfactant. For convenience reference is made to Remington: The Science and Practice of Pharmacy, 19th edition, 1995.

It is possible that other ingredients may be present in the peptide pharmaceutical formulation. Such additional ingredients may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical formulation.

Pharmaceutical compositions containing an antibody may be administered to a patient in need of such treatment at several sites, for example, at topical sites, for example, skin and mucosal sites, at sites which bypass absorption, for example, administration in an artery, in a vein, in the heart, and at sites which involve absorption, for example, administration in the skin, under the skin, in a muscle or in the abdomen. Administration of pharmaceutical compositions may be through several routes of administration, for example, subcutaneous, intramuscular, intraperitoneal, intravenous, lingual, sublingual, buccal, in the mouth, oral, in the stomach and intestine, nasal, pulmonary, for example, through the bronchioles and alveoli or a combination thereof, epidermal, dermal, transdermal, vaginal, rectal, ocular, for examples through the conjunctiva, uretal, and parenteral to patients in need of such a treatment.

### Diagnosis and treatment of malignancies

Methods of treating an individual, notably a human patient, using an anti-CD39 antibody as described herein are also provided for. In one embodiment, the disclosure provides for the use of an antibody as described herein in the preparation of a pharmaceutical composition for administration to a human patient. Typically, the patient suffers from, or is at risk for, cancer or an infectious disease (e.g., a viral infection, bacterial infection).

For example, in one aspect, provided is a method of restoring or potentiating the activity of lymphocytes in a patient in need thereof, comprising the step of administering a neutralizing anti-CD39 antibody of the disclosure to said patient.

In one embodiment, the method directed at increasing the activity of lymphocytes (e.g., T cells) in patients having a disease in which increased lymphocyte activity is beneficial or which is caused or characterized by immunosuppression, immunosuppressive cells, or, e.g., adenosine generated by CD4 T cells, CD8 T cells, B cells). The methods will be particularly useful for example patients having a solid tumor in which it is suspected the tumor microenvironment (and CD39-mediated adenosine production therein) may contribute to lack of recognition by the immune system (immune escape). The tumor may, for example, be characterized by CD39-expressing immune cells, e.g., CD4 T cells, CD8 T cells, B cells.

More specifically, the methods and compositions are utilized for the treatment of a variety of cancers and other proliferative diseases, and infectious diseases. Because these methods operate by reducing adenosine that inhibits the anti-target cell (e.g., anti-tumor) activity of lymphocytes and possibly additionally by increasing ATP that can increase the anti-tumor activity of lymphocytes, they are applicable to a very broad range of cancers and infectious disease. Representative examples of cancers that can be treated include in particular solid tumors in which adenosine in the tumor microenvironment may play a strong role in suppressing the anti-tumor immune response. In one embodiment, a human patient treated with an anti-CD39 antibody has liver cancer, bone cancer, pancreatic cancer, skin cancer, cancer of the head or neck, breast cancer, lung cancer, non- small cell lung cancer (NSCLC), castrate resistant prostate cancer (CRPC), melanoma, uterine cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B-cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B -lymphoblastic lymphoma, mantle cell lymphoma, acute lymphoblastic leukemia, mycosis fungoides, anaplastic large cell lymphoma, T-cell lymphoma, and precursor T-lymphoblastic lymphoma, and any combinations of said cancers. The present disclosure is also applicable to treatment of metastatic cancers. Patients can be tested or selected for one or more of the above described clinical attributes prior to, during or after treatment.

In one embodiment, the anti-CD39 antibody is administered in an amount effective to achieve and/or maintain in an individual (e.g., for 1, 2, 3, 4 weeks, and/or until the subsequent administration of antigen binding compound) a blood concentration of at least the EC₅₀, optionally the EC₇₀, optionally substantially the EC₁₀₀, for neutralization of the enzymatic activity of CD39. In one embodiment, the active amount of anti-CD39 antibody is an amount effective to achieve the EC₅₀, optionally the EC₇₀, optionally substantially the EC₁₀₀, for neutralization of the enzymatic activity of CD39 in an extravascular tissue of an individual. In one embodiment, the active amount of anti-CD39 antibody is an amount effective to achieve (or maintain) in an individual the EC₅₀, optionally the EC₇₀, optionally substantially the EC₁₀₀, for inhibition of neutralize the enzymatic activity of CD39.

Optionally, in one embodiment, in contrast to some antibodies that are directed to the depletion of CD39-expressing tumor cells by ADCC (which, e.g., can provide full efficacy at concentrations equal or substantially lower than that which provides receptor saturation), the anti-CD39 antibody does not exhibit substantial Fcγ receptor-mediated activity and is administered in an amount effective to neutralize the enzymatic activity of, optionally further CD39, without substantially causing down-modulation of CD39 expression, for a desired period of time, e.g., 1 week, 2 weeks, a month, until the next successive administration of anti-CD39 antibody.

In one embodiment, the anti-CD39 antibody is administered in an amount effective to achieve and/or maintain (e.g., for 1, 2, 3, 4 weeks, and/or until the subsequent administration of anti-CD39 antibody) in an individual a blood concentration of at least the EC₅₀, optionally the EC₇₀, optionally substantially the EC₁₀₀, for inhibition of CD39-mediated catabolism of ATP to AMP (e.g., by assessing neutralization of ATPase activity in B cells, optionally Ramos lymphoma cells, by quantifying hydrolysis of ATP to AMP). In one embodiment, the amount of anti-CD39 antibody is an amount effective to achieve (or maintain), in an extravascular tissue of an individual, the EC₅₀, optionally the EC₇₀, optionally substantially the EC₁₀₀, for inhibition of CD39-mediated catabolism of ATP to AMP.

In one embodiment, provided is a method for treating or preventing cancer in an individual, the method comprising administering to an individual having disease an anti-CD39 antibody in an amount that achieves or maintains for a specified period of time a concentration in circulation, optionally in an extravascular tissue of interest (e.g., the tumor or tumor environment), that is higher than the concentration required for 50%, 70%, or full (e.g., 90%) receptor saturation CD39-expressing cells in circulation (for example as assessed in PBMC). Optionally the concentration achieved is at least 20%, 50% or 100% higher than the concentration required for the specified receptor saturation.

In one embodiment, provided is a method for treating or preventing cancer in an individual, the method comprising administering to the individual an anti-CD39 antibody in an amount that achieves or maintains for a specified period of time a concentration in circulation, optionally in an extravascular tissue of interest (e.g., the tumor or tumor environment), that is higher than the EC₅₀, optionally EC₇₀ or optionally EC₁₀₀, for binding to CD39-expressing cells (e.g., as assessed by titrating anti-CD39 antibody on CD39-expressing cells, for example Ramos cells). Optionally the concentration achieved is at least 20%, 50% or 100% higher than the EC₅₀, optionally EC₇₀ or optionally EC₁₀₀, for binding to CD39-expressing cells.

The EC₅₀, EC₇₀ or the EC₁₀₀ can be assessed for example in a cellular assay for neutralization of the enzymatic activity of CD39 (e.g., neutralization of ATPase activity in Ramos cells by quantifying hydrolysis of ATP to AMP (or ATP to downstream adenosine). "EC₅₀" with respect to neutralization of the enzymatic activity of CD39, refers to the efficient concentration of anti-CD39 antibody which produces 50% of its maximum response or effect with respect to neutralization of the enzymatic activity. "EC₇₀" with respect to neutralization of the enzymatic activity of CD39, refers to the efficient concentration of anti-CD39 antibody which produces 70% of its maximum response or effect. "EC₁₀₀" with respect to neutralization of the enzymatic activity of CD39, refers to the efficient concentration of anti-CD39 antibody which produces its substantially maximum response or effect with respect to such neutralization of the enzymatic activity.

In some embodiments, particularly for the treatment of solid tumors, the concentration achieved is designed to lead to a concentration in tissues (outside of the vasculature, e.g., in the tumor or tumor environment) that corresponds to at least the EC₅₀ or EC₇₀ for neutralization of the enzymatic activity, optionally at about, or at least about, the EC₁₀₀.

In one embodiment, the amount of anti-CD39 antibody is between 1 and 20 mg/kg body weight. In one embodiment, the amount is administered to an individual weekly, every two weeks, monthly or every two months.

In one embodiment provided is a method of treating a human individual having a cancer, comprising administering to the individual an effective amount of an anti-CD39 antibody of the disclosure for at least one administration cycle (optionally at least 2, 3, 4 or more administration cycles), wherein the cycle is a period of eight weeks or less, wherein for each of the at least one cycles, one, two, three or four doses of the anti-CD39 antibody are administered at a dose of 1-20 mg/kg body weight. In one embodiment, the anti-CD39 antibody is administered by intravenous infusion.

Suitable treatment protocols for treating a human include, for example, administering to the patient an amount as disclosed herein of an anti-CD39 antibody, wherein the method comprises at least one administration cycle in which at least one dose of the anti-CD39 antibody is administered. Optionally, at least 2, 3, 4, 5, 6, 7 or 8 doses of the anti- CD39 antibody are administered. In one embodiment, the administration cycle is between 2 weeks and 8 weeks.

In one embodiment, provided is a method for treating or preventing a disease (e.g., a cancer, a solid tumor, a hematological tumor) in an individual, the method comprising administering to an individual having disease (e.g., a cancer, a solid tumor) an anti-CD39 antibody that neutralizes the enzymatic activity of CD39 for at least one administration cycle, the administration cycle comprising at least a first and second (and optionally a 3^{rd}, 4^{th}, 5^{th}, 6^{th}, 7^{th} and/or 8^{th} or further) administration of the anti-CD39 antibody, wherein the anti-CD39 antibody is administered in an amount effective to achieve, or to maintain between two successive administrations, a blood (serum) concentration of anti-CD39 antibody of at least 0.1 µg/ml, optionally at least 0.2 µg/ml, optionally at least 1 µg/ml, or optionally at least 2 µg/ml (e.g., for treatment of a hematological tumor), or optionally at least about 1 µg/ml, 2 µg/ml, 10 µg/ml, or 20 µg/ml, e.g., between 1-100 µg/ml, 1-50 µg/ml, 1-20 µg/ml, or 1-10 µg/ml (e.g., for treatment of a solid tumor, for treatment of a hematological tumor). In one embodiment, a specified continuous blood concentration is maintained, wherein the blood concentration does not drop substantially below the specified blood concentration for the duration of the specified time period (e.g., between two administrations of antibody, number of weeks, 1 week, 2 weeks, 3 weeks, 4 weeks), i.e. although the blood concentration can vary during the specified time period, the specified blood concentration maintained represents a minimum or "trough" concentration. In one embodiment, a therapeutically active amount of an anti-CD39 antibody is an amount of such antibody capable of providing (at least) the EC₅₀ concentration, optionally the EC₇₀ concentration optionally the EC₁₀₀ concentration, in blood and/or in a tissue for neutralization of the enzymatic activity of CD39 for a period of at least about 1 week, about 2 weeks, or about one month, following administration of the antibody.

Prior to or during a course of treatment with an anti-CD39 antibody of the disclosure, presence or levels or CD39-expressing cells, adenosine, ATP, ADP and/or AMP levels can be assessed within and/or adjacent to a patient's tumor to assess whether the patient is suitable for treatment (e.g., to predict whether the patient is likely to respond to treatment). Increased presence or levels or CD39-expressing cells, levels of adenosine, ATP, ADP and/or AMP may indicate an individual is suitable for treatment with (e.g., likely to benefit from) an anti-CD39 antibody of the disclosure (including but not limited to an antibody that inhibits substrate-bound CD39).

Prior to or during a course of treatment with an anti-CD39 antibody of the disclosure, adenosine, ADP and/or AMP levels can also be assessed within and/or adjacent to a patient's tumor to assess whether the patient is benefitting from treatment with an anti-CD39 antibody. Decreased levels of adenosine, ATP, ADP and/or AMP compared following an administration (or dosing of antibody) compared to levels prior to treatment (or dosing of antibody) may indicate an individual is benefitting from treatment with an anti-CD39 antibody of the disclosure (including but not limited to an antibody that inhibits substrate-bound CD39). Optionally, if a patient is benefitting from treatment with the anti-CD39 antibody, methods can further comprise administering a further dose of the anti-CD39 antibody to the patient (e.g., continuing treatment).

In one embodiment, assessing adenosine, ADP and/or AMP levels within and/or adjacent to a patient's tumor the tissue sample comprises obtaining from the patient a biological sample of a human tissue selected from the group consisting of tissue from a cancer patient, e.g., cancer tissue, tissue proximal to or at the periphery of a cancer, cancer adjacent tissue, adjacent non-tumorous tissue or normal adjacent tissue, and detecting adenosine, ATP, ADP and/or AMP levels within the tissue. The levels from the patient can be comparing the level to a reference level, e.g., corresponding to a healthy individual.

In one embodiment, the disclosure provides a method for the treatment or prevention of a cancer in an individual in need thereof, the method comprising:
a) detecting CD39-expressing cells (or adenosine, ATP, ADP and/or AMP) in the tumor environment, optionally within the tumor and/or within adjacent tissue, and
b) upon a determination that tumor environment comprises CD39-expressing cells (or adenosine, ATP, ADP and/or AMP), optionally at a level that is increased compared to a reference level (e.g., corresponding to a healthy individual or an individual not deriving substantial benefit from an anti-CD39 antibody), administering to the individual an anti-CD39 antibody.

In one embodiment, the disclosure provides a method for the treatment or prevention of a cancer in an individual in need thereof, the method comprising:
a) detecting cells in circulation that express vascular CD39 (e.g., from a blood sample), and
b) upon a detection of cells in circulation that express vascular CD39, optionally at a level that is increased compared to a reference level (e.g., corresponding to a healthy individual or an individual not deriving substantial benefit from an anti-CD39 antibody), administering to the individual an anti-CD39 antibody.

In one embodiment, the disclosure provides a method for the treatment or prevention of a cancer in an individual in need thereof, the method comprising:
a) detecting cells that express vascular CD39 in the tumor environment, optionally within the tumor and/or within adjacent tissue, and
b) upon a detection of cells in the tumor environment that express vascular CD39, optionally at a level that is increased compared to a reference level (e.g., corresponding to a healthy individual or an individual not deriving substantial benefit from an anti-CD39 antibody), administering to the individual an anti-CD39 antibody.

Optionally, in any of the methods, detecting CD39-expressing cells (or adenosine, ATP, ADP and/or AMP) within the tumor environment comprises obtaining from the individual a biological sample that comprises cancer tissue and/or tissue proximal to or at the periphery of a cancer (e.g., cancer adjacent tissue, adjacent non-tumorous tissue or normal adjacent tissue), and detecting levels of CD39-expressing cells (or adenosine, ATP, ADP and/or AMP). CD39-expressing cells may comprise, for example, tumor cells, CD4 T cells, CD8 T cells, TReg cells, B cells.

A patient having a cancer can be treated with the anti-CD39 antibody with our without a prior detection step to assess expression of CD39 on cells in the tumor microenvironment (e.g., on tumor cells, CD4 T cells, CD8 T cells, TReg cells, B cells). Optionally, the treatment methods can comprises a step of detecting a CD39 nucleic acid or polypeptide in a biological sample of a tumor from an individual (e.g., in cancer tissue, tissue proximal to or at the periphery of a cancer, cancer adjacent tissue, adjacent non-tumorous tissue or normal adjacent tissue). A determination that a biological sample comprises cells expressing CD39 (e.g., prominently expressing; expressing CD39 at a high level, high intensity of staining with an anti-CD39 antibody, compared to a reference) indicates that the patient has a cancer that may have a strong benefit from treatment with an agent that inhibits CD39. In one embodiment, the method comprises determining the level of expression of a CD39 nucleic acid or polypeptide in a biological sample and comparing the level to a reference level corresponding to a healthy individual. A determination that a biological sample comprises cells expressing CD39 nucleic acid or polypeptide at a level that is increased compared to the reference level indicates that the patient has a cancer that can be treated with an anti-CD39 antibody of the disclosure. Optionally, detecting a CD39 polypeptide in a biological sample comprises detecting CD39 polypeptide expressed on the surface of a malignant cell, or of a CD4 T cell, CD8 T cell, TReg cell or B cell. In one embodiment, a determination that a biological sample comprises cells that prominently expresses CD39 nucleic acid or polypeptide indicates that the patient has a cancer that can be treated with an anti-CD39 antibody of the disclosure. "Prominently expressed", when referring to a CD39 polypeptide, means that the CD39 polypeptide is expressed in a substantial number of cells taken from a given patient. While the definition of the term "prominently expressed" is not bound by a precise percentage value, in some examples a receptor said to be "prominently expressed" will be detected on at least 10%, 20% 30%, 40%, 50%, 60%, 70%, 80%, or more of the tumor cells taken from a patient.

Determining whether an individual has a cancer characterized by cells that express a CD39 polypeptide can for example comprise obtaining a biological sample (e.g., by performing a biopsy) from the individual that comprises cells from the cancer environment (e.g., tumor or tumor adjacent tissue), bringing said cells into contact with an antibody that binds an CD39 polypeptide, and detecting whether the cells express CD39 on their surface. Optionally, determining whether an individual has cells that express CD39 comprises conducting an immunohistochemistry assay.

In one embodiment, the disclosure provides a method for the treatment or prevention of a cancer in an individual in need thereof, the method comprising:
a) determining the CD39 polypeptide status of cells within the tumor environment, optionally within the tumor and/or within adjacent tissue, and
b) upon a determination that tumor environment comprises cells that express CD39 polypeptide, optionally at a level that is increased compared to a reference level, administering to the individual an anti-CD39 antibody. In one embodiment, the cells are tumor cells. In another embodiment, the cells within the tumor environment, tumor and/or adjacent tissue are non-malignant immune cells, e.g., B cells, T cells, TReg cells. Optionally, determining the CD39 polypeptide status within the tumor environment comprises obtaining from the individual a biological sample that comprises cancer tissue and/or tissue proximal to or at the periphery of a cancer (e.g., cancer adjacent tissue, adjacent non-tumorous tissue or normal adjacent tissue), bringing said cells into contact with an antibody that binds a CD39 polypeptide, and detecting cells that express CD39.

The antibody compositions may be used in as monotherapy or combined treatments with one or more other therapeutic agents, including agents normally utilized for the particular therapeutic purpose for which the antibody is being administered. The additional therapeutic agent will normally be administered in amounts and treatment regimens typically used for that agent in a monotherapy for the particular disease or condition being treated. Such therapeutic agents include, but are not limited to anti-cancer agents and chemotherapeutic agents.

In one embodiment, the second or additional second therapeutic agent is an antibody or other Fc domain-containing protein capable of inducing ADCC toward a cell to which it is bound, e.g., via CD16 expressed by an NK cell. Typically, such antibody or other protein will comprise a domain that binds to an antigen of interest, e.g., an antigen present on a tumor cell (tumor antigen), and an Fc domain or portion thereof, and will exhibit binding to the antigen via the antigen binding domain and to Fcγ receptors (e.g., CD16) via the Fc domain. In one embodiment, its ADCC activity will be mediated at least in part by CD16. In one embodiment, the additional therapeutic agent is an antibody having a native or modified human Fc domain, for example a Fc domain from a human IgG1 or IgG3 antibody. The term "antibody-dependent cell-mediated cytotoxicity" or "ADCC" is a term well understood in the art, and refers to a cell-mediated reaction in which non-specific cytotoxic cells that express Fc receptors (FcRs) recognize bound antibody on a target cell and subsequently cause lysis of the target cell. Non-specific cytotoxic cells that mediate ADCC include natural killer (NK) cells, macrophages, monocytes, neutrophils, and eosinophils. The term "ADCC-inducing antibody" refers to an antibody that demonstrates ADCC as measured by assay(s) known to those of skill in the art. Such activity is typically characterized by the binding of the Fc region with various FcRs. Without being limited by any particular mechanism, those of skill in the art will recognize that the ability of an antibody to demonstrate ADCC can be, for example, by virtue of it subclass (such as IgG1 or IgG3), by mutations introduced into the Fc region, or by virtue of modifications to the carbohydrate patterns in the Fc region of the antibody.

In the treatment methods, the CD39-binding compound and the second therapeutic agent can be administered separately, together or sequentially, or in a cocktail. In some embodiments, the antigen-binding compound is administered prior to the administration of the second therapeutic agent. For example, the CD39-binding compound can be administered approximately 0 to 30 days prior to the administration of the second therapeutic agent. In some embodiments, an CD39-binding compound is administered from about 30 minutes to about 2 weeks, from about 30 minutes to about 1 week, from about 1 hour to about 2 hours, from about 2 hours to about 4 hours, from about 4 hours to about 6 hours, from about 6 hours to about 8 hours, from about 8 hours to 1 day, or from about 1 to 5 days prior to the administration of the second therapeutic agent. In some embodiments, a CD39-binding compound is administered concurrently with the administration of the therapeutic agents. In some embodiments, a CD39-binding compound is administered after the administration of the second therapeutic agent. For example, a CD39-binding compound can be administered approximately 0 to 30 days after the administration of the second therapeutic agent. In some embodiments, a CD39-binding compound is administered from about 30 minutes to about 2 weeks, from about 30 minutes to about 1 week, from about 1 hour to about 2 hours, from about 2 hours to about 4 hours, from about 4 hours to about 6 hours, from about 6 hours to about 8 hours, from about 8 hours to 1 day, or from about 1 to 5 days after the administration of the second therapeutic agent.

### Examples

### Example 1: Production of modified anti-CD39 antibodies

A parental murine antibody having strong ability to inhibit human CD39 was found to have relatively low physicochemical stability. Heavy and light chain variable regions were designed that incorporated the CDRs from the murine parental antibody and human framework regions, taking into consideration potential amino acid modifications. 3D models based on different human VH gene segments were superimposed and all amino acid differences from the murine parental antibody were scrutinized one by one. A salt bridge between Abnum position residues E46 and R38/Q43 (E46 forms H-bonds with R38 and Q43) was introduced through the use of a framework 2 (FR2) of the IGHV7-4-1*01 gene segment. Intrachain and extrachain connections between residues were assessed by modelling, showing that in antibodies having a glutamic acid at Abnuum position 46 in the heavy chain, the E46 forms H-bonds with R38 and Q43, and D62 (CDR2) is not engaged, as shown below:

|--------|
|--|
FTHYGMNWVRQAPGQGLEWMGWINTYTGEPTYADDFK
(SEQ ID NO: 34)

A further modification to the FR2 provided variants in which a salt bridge was introduced between the framework 2 (FR2) and CDR2 by placing a lysine at residue 46 (E46K substitution). The D62 loop is therefore slightly displaced (less rigid) when a glutamic acid is present at position 46 compared to when a lysine is present at K46. D62 is inside the CDR-H2, consequently we repositioned CDR2 (residue D62) via the salt bridge with K46 by removing the salt bridges between R38 and/or Q43 and introducing a salt bridge between residue at Abnum positions 46 and 62, as shown below:

|-----------------|

FTHYGMNWVRQAPGQGLKWMGWINTYTGEPTYADDFK

(SEQ ID NO: 35)

The amino acid sequences of respective heavy and light chain variable regions are shown below (residues differing between sequence underlined).
mAb1 
VH:

QVQLVQSGSELKKPGASVKVSCKASGYTFTHYGMNWVRQAPGQGLEWMGWINTYTGEPTYAD

DFKGRFVFSLDTSVSTAYLQISSLKAEDTAVYYCARRRYEGNYVFYYFDYWGQGTTVTVSS

(SEQ ID NO: 6)
 
VL:
DIQMTQSPSSLSASVGDRVTITCRASENIYSYFSWYQQKPGKAPKLLIYTAKTLAEGVPSRFSGSGSG
TDFTLTISSLQPEDFATYYCQHHYVTPYTFGGGTKVEIK
(SEQ ID NO: 7)
mAb2 
VH:

QVQLVQSGSELKKPGASVKVSCKASGYTFTHYGMNWVRQAPGQGLKWMGWINTYTGEPTYADDFKGRF

VFSLDTSVSTAYLQISSLKAEDTAVYYCARRRYEGNYVFYYFDYWGQGTTVTVSS

(SEQ ID NO: 8)
 
VL:
DIQMTQSPSSLSASVGDRVTITCRASENIYSYFSWYQQKPGKAPKLLIYTAKTLAEGVPSRFSGSGSG
TDFTLTISSLQPEDFATYYCQHHYVTPYTFGGGTKVEIK
(SEQ ID NO: 9)
mAb3 
VH:
QVQLVQSGSELKKPGASVKVSCKASGYTFTHYGMNWVRQAPGKGLKWMGWINTYTGEPTYADDFKGRF
VFSLDTSVSTAYLQISSLKAEDTAVYYCARRRYEGNYVFYYFDYWGQGTTVTVSS
(SEQ ID NO: 10)
 
VL:
DIQMTQSPSSLSASVGDRVTITCRASENIYSYFSWYQQKPGKAPKLLIYTAKTLAEGVPSRFSGSGSG
TDFTLTISSLQPEDFATYYCQHHYVTPYTFGGGTKVEIK
(SEQ ID NO: 11)
mAb4 
VH:
QIQLVQSGSELKKPGASVKVSCKASGYTFTHYGMNWVRQAPGKGLKWMGWINTYTGEPTYADDFKGRF
VFSLDASVSTAYLQISSLKAEDTAVYYCARRRYEGNYVFYYFDYWGQGTTVTVSS
(SEQ ID NO: 12)
 
VL:
DIQMTQSPSSLSASVGDRVTITCRASENIYSYFSWYQQKPGKAPKLLIYTAKTLAEGVPSRFSGSGSG
TDFTLTISSLQPEDFATYYCQHHYVTPYTFGGGTKVEIK
(SEQ ID NO: 13)
mAb5 
VH:
QVQLVQSGSELKKPGASVKVSCKASGYTFTHYGMNWVRQAPGQGLKWMGWINTYTGEPTYADDFKGRF
VFSLDTSVSTAYLQISSLKAEDTAVYYCARRRYEGNYVFYYFDYWGQGTTVTVSS
(SEQ ID NO: 14)
 
VL:
DIQMTQSPSSLSASVGDRVTITCRASENIYSYFSWYQQKPGKAPKLLVYTAKTLAEGVPSRFSGSGSG
TDFTLTISSLQPEDFATYYCQHHYVTPYTFGGGTKVEIK
(SEQ ID NO: 15)
mAb6 
VH:

QVQLVQSGSELKKPGASVKVSCKASGYTFTHYGMNWVRQAPGKGLKWMGWINTYTGEPTYADDFKGRF

VFSLDTSVSTAYLQISSLKAEDTAVYYCARRRYEGNYVFYYFDYWGQGTTVTVSS

(SEQ ID NO: 16)
 
VL:

DIQMTQSPSSLSASVGDRVTITCRASENIYSYFSWYQQKPGKAPKLLVYTAKTLAEGVPSRFSGSGSG

TDFTLTISSLQPEDFATYYCQHHYVTPYTFGGGTKVEIK

(SEQ ID NO: 17)
mAb7

(SEQ ID NO: 18)

VH:

QIQLVQSGSELKKPGASVKVSCKASGYTFTHYGMNWVRQAPGKGLKWMGWINTYTGEPTYADDFKGRF

VFSLDASVSTAYLQISSLKAEDTAVYYCARRYEGNYVFYYFDYWGQGTTVTVSS

(SEQ ID NO: 19)
VL:
DIQMTQSPSSLSASVGDRVTITCRASENIYSYFSWYQQKPGKAPKLLVYTAKTLAEGVPSRFSGSGSG
TDFTLTISSLQPEDFATYYCQHHYVTPYTFGGGTKVEIK

The antibodies having the VH and Vk variable regions shown were produced as Fc silent recombinant chimeric human IgG1 antibodies with a heavy chain N297Q (Kabat EU numbering) mutation which results in lack of N-linked glycosylation and loss of binding to human Fcγ receptors CD16A, CD16B, CD32A, CD32B and CD64.

Briefly, the VH and Vk sequences of each antibody were cloned into vectors containing the hulgG1 constant domains (harbouring the N297Q mutation) and the huCk constant domain respectively. The two obtained vectors were co-transfected into the CHO cell line. The established pool of cell was used to produce the antibody in the CHO medium. The antibody was then purified using protein A.

### Example 2: ELISA titration on CD39-L1, L2, L3, L4 isoforms

Antibodies were tested for binding to recombinant human CD39 isoforms (RechuCD39 isoforms). The CD39 isoforms having amino acid sequences shown in the table below were coated in 96-well plate in PBS 1X at 500ng/ml or 1µg/ml at 4°C overnight. Wells were washed in TBS Tween 20, and further saturated 2H at RT in TBS Blocking buffer (Thermo Fisher Scientific Inc.). Dose range concentration of primary antibody was incubated in TBS blocking buffer for 2h at RT. Wells were washed in TBS Tween 20. Secondary Antibody (GAM-HRP or GAH-HRP in TBS blocking buffer) was incubated for 1H at RT, and was revealed with TMB (Interchim, France). Optical density was measured on Enspire at OD=450.

### Amino acid sequence of the cloned huCD39 (vascular isoform):

Human CD39-L1, also known as NTPDase2 or ENTPD2: 
1 MAGKVRSLLP PLLLAAAGLA GLLLLCVPTR DVREPPALKY GIVLDAGSSH TSMFIYKWPA
61 DKENDTGIVG QHSSCDVPGG GISSYADNPS GASQSLVGCL EQALQDVPKE RHAGTPLYLG
121 ATAGMRLLNL TNPEASTSVL MAVTHTLTQY PFDFRGARIL SGQEEGVFGW VTANYLLENF
181 IKYGWVGRWF RPRKGTLGAM DLGGASTQIT FETTSPAEDR ASEVQLHLYG QHYRVYTHSF
241 LCYGRDQVLQ RLLASALQTH GFHPCWPRGF STQVLLGDVY QSPCTMAQRP QNFNSSARVS
301 LSGSSDPHLC RDLVSGLFSF SSCPFSRCSF NGVFQPPVAG NFVAFSAFFY TVDFLRTSMG
361 LPVATLQQLE AAAVNVCNQT WAQQLLSRGY GFDERAFGGV IFQKKAADTA VGWALGYMLN
421 LTNLIPADPP GLRKGTDFSS WVVLLLLFAS ALLAALVLLL RQVHSAKLPS TI
(SEQ ID NO: 2)
Human CD39-L2, also known as NTPDase6 or ENTPD6: 
1 MKKGIRYETS RKTSYIFQQP QHGPWQTRMR KISNHGSLRV AKVAYPLGLC VGVFIYVAYI
61 KWHRATATQA FFSITRAAPG ARWGQQAHSP LGTAADGHEV FYGIMFDAGS TGTRVHVFQF
121 TRPPRETPTL THETFKALKP GLSAYADDVE KSAQGIRELL DVAKQDIPFD FWKATPLVLK
181 ATAGLRLLPG EKAQKLLQKV KEVFKASPFL VGDDCVSIMN GTDEGVSAWI TINFLTGSLK
241 TPGGSSVGML DLGGGSTQIA FLPRVEGTLQ ASPPGYLTAL RMFNRTYKLY SYSYLGLGLM
301 SARLAILGGV EGQPAKDGKE LVSPCLSPSF KGEWEHAEVT YRVSGQKAAA SLHELCAARV
361 SEVLQNRVHR TEEVKHVDFY AFSYYYDLAA GVGLIDAEKG GSLVVGDFEI AAKYVCRTLE
421 TQPQSSPFSC MDLTYVSLLL QEFGFPRSKV LKLTRKIDNV ETSWALGAIF HYIDSLNRQK
481 spas
(SEQ ID NO: 3)
Human CD39-L3, also known as NTPDase3 or ENTPD3: 
1 MFTVLTRQPC EQAGLKALYR TPTIIALVVL LVSIVVLVSI TVIQIHKQEV LPPGLKYGIV
61 LDAGSSRTTV YVYQWPAEKE NNTGVVSQTF KCSVKGSGIS SYGNNPQDVP RAFEECMQKV
121 KGQVPSHLHG STPIHLGATA GMRLLRLQNE TAANEVLESI QSYFKSQPFD FRGAQIISGQ
181 EEGVYGWITA NYLMGNFLEK NLWHMWVHPH GVETTGALDL GGASTQISFV AGEKMDLNTS
241 DIMQVSLYGY VYTLYTHSFQ CYGRNEAEKK FLAMLLQNSP TKNHLTNPCY PRDYSISFTM
301 GHVFDSLCTV DQRPESYNPN DVITFEGTGD PSLCKEKVAS IFDFKACHDQ ETCSFDGVYQ
361 PKIKGPFVAF AGFYYTASAL NLSGSFSLDT FNSSTWNFCS QNWSQLPLLL PKFDEVYARS
421 YCFSANYIYH LFVNGYKFTE ETWPQIHFEK EVGNSSIAWS LGYMLSLTNQ IPAESPLIRL
481 PIEPPVFVGT LAFFTAAALL CLAFLAYLCS ATRRKRHSEH AFDHAVDSD
(SEQ ID NO: 4)
Human CD39-L4, also known as NTPDase5 or ENTPD5:

1 MATSWGTVFF MLVVSCVCSA VSHRNQQTWF EGIFLSSMCP INVSASTLYG IMFDAGSTGT
61 RIHVYTFVQK MPGQLPILEG EVFDSVKPGL SAFVDQPKQG AETVQGLLEV AKDSIPRSHW
121 KKTPVVLKAT AGLRLLPEHK AKALLFEVKE IFRKSPFLVP KGSVSIMDGS DEGILAWVTV
181 NFLTGQLHGH RQETVGTLDL GGASTQITFL PQFEKTLEQT PRGYLTSFEM FNSTYKLYTH
241 SYLGFGLKAA RLATLGALET EGTDGHTFRS ACLPRWLEAE WIFGGVKYQY GGNQEGEVGF
301 EPCYAEVLRV VRGKLHQPEE VQRGSFYAFS YYYDRAVDTD MIDYEKGGIL KVEDFERKAR
361 EVCDNLENFT SGSPFLCMDL SYITALLKDG FGFADSTVLQ LTKKVNNIET GWALGATFHL
421 LQSLGISH
(SEQ ID NO: 5)

The anti-CD39 antibodies mAbs1-7 bound to the vascular CD39 but not to any of the CD39 isoforms CD39-L1, -L2, -L3 or-L4.

### Example 3: Assessment by Maldi TOF of neutralization of ATPase activity

The inhibition of CD39 enzymatic activity by antibodies was evaluated by Maldi TOF mass spectrometry (production of AMP). This assay represents an assay that is relatively insensitive to down-modulation of CD39 expression. Briefly, 10⁵ Ramos human lymphoma cells were incubated overnight at 37°C with anti-CD39 antibodies or a chemical CD39 inhibitor (ARL 100 µM). After washes, cells were incubated with anti-CD39 antibodies, isotype control, or ARL and 50 µM ATP for 30 minutes at 4°C in PBS. AMP generated was quantified in supernatants by Maldi TOF. The anti-CD39 antibodies mAbs1-7 were highly potent at blocking CD39 enzymatic activity, providing for inhibition of at least about 90% of enzymatic activity.

### Example 4: Ab titration on Ramos tumor cells by flow cytometry

Ramos human lymphoma cells that endogenously express CD39 were used to evaluate ability of different anti-CD39 antibodies to bind human CD39. Briefly, 10⁵ Ramos cells were resuspended in PBS/0.2% BSA/0.02% NaN3 (named "staining buffer") are distributed into round bottom 96W-microplates. Dose-range of anti-CD39 antibodies was added to the plates and cells are incubated for 45 min at 4°C. Cells were washed three times in staining buffer by spinning plates at 400 g for 3 min at 4°C. PE-coupled goat anti-mouse or goat anti-human IgG Fc fragment secondary antibodies (Beckman Coulter) diluted in staining buffer were added to the cells and plates are incubated for 30 additional minutes at 4°C. Cells were washed three times as described above and analyzed on an Accury C6 flow cytometer equipped with an HTFC plate reader.

Median of fluorescence vs. antibodies concentration was plotted on graphs and EC50 is calculated using GraphPad Prism program.

Results showed that each antibody showed strong binding to Ramos cells.

### Example 5: Increased physicochemical stability of antibodies

The anti-CD39 antibodies modified by introduction of human VH and VL acceptor frameworks to introduce a salt bridge in the VH were produced as a human IgG1 in a variety of different variants having different mutations in the heavy chain constant regions that each caused a reduction and/or loss of binding to human Fc receptors while retaining CD39 binding. The VH and Vk sequences of mAb2 of Example 1 antibody were cloned into vectors containing the hulgG1 CH1 constant domain and the huCk constant domain respectively. The two obtained vectors were co-transfected into the CHO cell line.

The resulting antibodies had the heavy and light chain amino acid sequences shown below:

### Heavy chain amino acid sequences

*L234F*/*L235E*/*P331S mutation:* 
QVQLVQSGSELKKPGASVKVSCKASGYTFTHYGMNWVRQAPGQGLKWMGWINTYTGEPTYADDFKGRF
VFSLDTSVSTAYLQISSLKAEDTAVYYCARRRYEGNYVFYYFDYWGQGTTVTVSSASTKGPSVFPLAP
SSKSTSGGTAALGCLVKDYFPEPVTVSWNSGALTSGVHTFPAVLQSSGLYSLSSVVTVPSSSLGTQTY
ICNVNHKPSNTKVDKRVEPKSCDKTHTCPPCPAPEFEGGPSVFLFPPKPKDTLMISRTPEVTCVVVDV
SHEDPEVKFNWYVDGVEVHNAKTKPREEQYNSTYRVVSVLTVLHQDWLNGKEYKCKVSNKALPASIEK
TISKAKGQPREPQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 26)
*L234A*/*L235E*/*P331S mutation:* 
QVQLVQSGSELKKPGASVKVSCKASGYTFTHYGMNWVRQAPGQGLKWMGWINTYTGEPTYADDFKGRF
VFSLDTSVSTAYLQISSLKAEDTAVYYCARRRYEGNYVFYYFDYWGQGTTVTVSSASTKGPSVFPLAP
SSKSTSGGTAALGCLVKDYFPEPVTVSWNSGALTSGVHTFPAVLQSSGLYSLSSVVTVPSSSLGTQTY
ICNVNHKPSNTKVDKRVEPKSCDKTHTCPPCPAPEAEGGPSVFLFPPKPKDTLMISRTPEVTCVVVDV
SHEDPEVKFNWYVDGVEVHNAKTKPREEQYNSTYRVVSVLTVLHQDWLNGKEYKCKVSNKALPASIEK
TISKAKGQPREPQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 27)
L234A/L235E/G237A/A330S/P331S mutation: 
QVQLVQSGSELKKPGASVKVSCKASGYTFTHYGMNWVRQAPGQGLKWMGWINTYTGEPTYADDFKGRF
VFSLDTSVSTAYLQISSLKAEDTAVYYCARRRYEGNYVFYYFDYWGQGTTVTVSSASTKGPSVFPLAP
SSKSTSGGTAALGCLVKDYFPEPVTVSWNSGALTSGVHTFPAVLQSSGLYSLSSVVTVPSSSLGTQTY
ICNVNHKPSNTKVDKRVEPKSCDKTHTCPPCPAPEAEGAPSVFLFPPKPKDTLMISRTPEVTCVVVDV
SHEDPEVKFNWYVDGVEVHNAKTKPREEQYNSTYRVVSVLTVLHQDWLNGKEYKCKVSNKALPSSIEK
TISKAKGQPREPQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 28)
L234A/L235E/G237A/P331S mutation: 
QVQLVQSGSELKKPGASVKVSCKASGYTFTHYGMNWVRQAPGQGLKWMGWINTYTGEPTYADDFKGRF
VFSLDTSVSTAYLQISSLKAEDTAVYYCARRRYEGNYVFYYFDYWGQGTTVTVSSASTKGPSVFPLAP
SSKSTSGGTAALGCLVKDYFPEPVTVSWNSGALTSGVHTFPAVLQSSGLYSLSSVVTVPSSSLGTQTY
ICNVNHKPSNTKVDKRVEPKSCDKTHTCPPCPAPEAEGAPSVFLFPPKPKDTLMISRTPEVTCVVVDV
SHEDPEVKFNWYVDGVEVHNAKTKPREEQYNSTYRVVSVLTVLHQDWLNGKEYKCKVSNKALPASIEK
 
TISKAKGQPREPQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 29)
Light chain amino acid sequence

DIQMTQSPSSLSASVGDRVTITCRASENIYSYFSWYQQKPGKAPKLLIYTAKTLAEGVPSRFSGSGSG
TDFTLTISSLQPEDFATYYCQHHYVTPYTFGGGTKVEIKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLSSP
VTKSFNRGEC (SEQ ID NO: 9)

The resulting antibodies were tested for stability in the following reference formulation at a concentration of approximatively 7mg/mL: pH 6.0; histidine buffer (10mM); sucrose (200mM); NaCl (50mM); Polysorbate 80 (PS80) (0.2g/L). A high concentration of PS80 was tested separately at 0.5g/L, however this did not permit a reduction in the macroscopic aggregation of the antibody. So for this study the concentration is set at 0.2g/L. The stability of the formulations was monitored in two storage conditions (at +5°C± 3°C and at +40 ± 3°C. For each study, 3 times point were performed: T0, T15D (15 days) and T1M (1 month). A freeze thaw (F/T) and a thermal shift stability assay (TSSA) were conducted for the format comparison. To perform F/T cycles, the samples were frozen at least 2 hours at -20°C and thawed at least 1 hour at room temperature, the F/T cycle is repeated three times and samples are tested 24h after the last Freeze/Thaw cycle. At each time point, the following tests were performed:
- Particulate Matter (MFI)
- Visual Inspection (Appearance)
- Impurities (SE-HPLC)
- Turbidity (400nm)
- Protein Concentration (280nm) (performed with Nanodrop, Thermo Fisher Scientific Inc.)

The resulting antibodies showed good physicochemical stability. The parental (murine) antibody displays a relatively low inherent stability which is believed to be due to the numerous aromatic amino acid residues at the surface of the mAb, located in the CDRs, particularly in heavy chain CDR3. These aromatic residues confer a relatively high predicted hydrophobicity to the antibody. However, the aromatic residues in these antibodies are believed to be important for antibody function and can probably not be replaced by a non-aromatic residue without a reduction or loss of activity. Interestingly, several mutants showed a higher aggregation temperature (TAgg) compared to the N297Q mutant of human IgG1, as shown in the table below. Aggregation temperature is correlated with the intrinsic stability. The higher the TAgg, the higher the stability of the protein. Surprisingly, Fc domain variants with mutations in the hinge displayed highest stability, and moreover improved the stability of the antibody compared to the N297Q variant, and furthermore stability was improved compared to the parental mouse antibody and to the antibody as a human IgG4, the latter displaying particularly low stability (aggregation). The stability of each of the Fc domain of human IgG1 isotype comprises an amino acid substitution at Kabat residue(s) 234, 235, 237, 330 and/or 331 (L234A/L235E/P331S substitutions, L234F/L235E/P331S substitutions, L234A/L235E/G237A/P331S substitutions, and L234A/L235E/G237A/A330S/P331S substitutions) improved the antibody, as shown in the table below. Such mutations can therefore enhance the pharmacological properties and/or activity of the antibody.

| **Format (human IgG1 Fc mutations)** | **T_{agg} run1** | **T_{agg} run2** | **T_{agg} run3** | **SD** | **T_{agg} Mean** |
|---|---|---|---|---|---|
| L234F/L235E/P331S | 67.65 | 67.87 | 68.34 | 0.35 | 68.0 |
| L234A/L235E/P331S | 66,50 | 66.91 | 67.77 | 0.65 | 67.1 |
| L234A/L235E/G237A/A330S/P331S | 66.35 | 67.58 | 67.07 | 0.62 | 67.0 |
| L234A/L235E/G237A/ P331S | 66.08 | 66.55 | 66.29 | 0.24 | 66.3 |
| N297Q | 63.41 | 62.91 | 63.81 | 0.45 | 63.4 |

| | | | | | |
|---|---|---|---|---|---|
| SD = Standard Deviation TAgg = Temperature of Aggregation | | | | | |

The parental antibody displays a relatively low inherent stability which is believed to be due to the numerous aromatic amino acid residues at the surface of the mAb, located in the CDRs. The antibody bears an unusually high number of aromatic acid residues in their CDRs (particularly in CDR-H3). These aromatic residues confer a relatively high predicted hydrophobicity to the antibody. However, the aromatic residues in these antibodies are likely to be important for antibody function, as several of the residues are involved in important interactions with CD39 and/or VH-VL interactions and can probably not be replaced by a non-aromatic residue without a reduction or loss of activity. The ability to enhance the physicochemical stability is valuable for pharmaceutical use.

All references, including publications, patent applications, and patents, cited herein are hereby incorporated by reference in their entirety and to the same extent as if each reference were individually and specifically indicated to be incorporated by reference and were set forth in its entirety herein (to the maximum extent permitted by law), regardless of any separately provided incorporation of particular documents made elsewhere herein.

The use of the terms "a" and "an" and "the" and similar references are to be construed to cover both the singular and the plural, unless otherwise indicated herein or clearly contradicted by context.

Unless otherwise stated, all exact values provided herein are representative of corresponding approximate values (e.g., all exact exemplary values provided with respect to a particular factor or measurement can be considered to also provide a corresponding approximate measurement, modified by "about," where appropriate).

The description herein of any aspect or embodiment herein using terms such as "comprising", "having," "including," or "containing" with reference to an element or elements is intended to provide support for a similar aspect or embodiment herein that "consists of", "consists essentially of", or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

In one embodiment (I), provided is an antibody that binds a human CD39 polypeptide expressed by a cell and that neutralizes the ATPase activity thereof, selected from the group consisting of:
(a) an antibody comprising a heavy chain comprising an amino acid sequence of SEQ ID NO: 26 and a light chain comprising an amino acid sequence of SEQ ID NO: 9;
(b) an antibody comprising a heavy chain comprising an amino acid sequence of SEQ ID NO: 27 and a light chain comprising an amino acid sequence of SEQ ID NO: 9;
(c) an antibody comprising a heavy chain comprising an amino acid sequence of SEQ ID NO: 28 and a light chain comprising an amino acid sequence of SEQ ID NO: 9; and
(d) an antibody comprising a heavy chain comprising an amino acid sequence of SEQ ID NO: 29 and a light chain comprising an amino acid sequence of SEQ ID NO: 9.

In another embodiment (II), provided is an antibody that binds a human CD39 polypeptide expressed by a cell and that neutralizes the ATPase activity thereof, comprising a heavy chain variable region (VH) comprising a CDR1, CDR2 and CDR3 having the respective amino acid sequences shown in SEQ ID NOS: 20, 21 and 22 and a light chain variable region (VL) comprising a CDR1, CDR2 and CDR3 having the respective amino acid sequences shown in SEQ ID NOS: 23, 24 and 25, wherein the VH comprises an amino acid sequence at least 90%, 95% or 98% identical to the amino acid sequence of SEQ ID NO: 6, and the VL comprises an amino acid sequence at least 90%, 95% or 98% identical to the amino acid sequence of SEQ ID NO: 7.

According to a first particular embodiment of (II),
the heavy chain variable region (VH) comprises an arginine (R) amino acid residue at position 38 and/or a glutamine (Q) amino acid residue at position 43, a glutamic acid (E) amino acid residue at position 46 and an aspartic acid (D) amino acid residue at position 62, wherein numbering is according to Abnum, and/or
the VH comprises an amino acid sequence of SEQ ID NO: 6 and the VL comprises an amino acid sequence of SEQ ID NO: 7.

According to a second particular embodiment of (II),
the heavy chain variable region (VH) comprises a lysine (K) amino acid residue at position 46 and an aspartic acid (D) amino acid residue at position 62, wherein numbering is according to Abnum,
advantageously the heavy chain variable region (VH) comprises an arginine (R) amino acid residue at position 38 and/or a glutamine (Q) amino acid residue at position 43, a lysine (K) amino acid residue at position 46 and an aspartic acid (D) amino acid residue at position 62, wherein numbering is according to Abnum.

According to embodiment (II) and the second particular embodiments of (II), the VH may comprise an amino acid sequence of SEQ ID NO: 8 and the VL may comprise an amino acid sequence of SEQ ID NO: 9.

According to any of the previous embodiments of (II), the VH human acceptor framework may be from IGHV7-4-1 and the J-segment may be from IGHJ6.

According to any of the previous embodiments of (II), the VL domain human acceptor framework may be from IGKJ4 or IGKJ2.

According to any of the previous embodiments of (II), the VH domain may comprise a FR2-CDR2 segment comprising the amino acid sequence of SEQ ID NO: 35.

In a further embodiment (III), provided is an antibody that binds human CD39 and that neutralizes the ATPase activity thereof, selected from the group consisting of:
(a) an antibody comprising a heavy chain variable region comprising an amino acid sequence of SEQ ID NO: 6 and a light chain variable region comprising an amino acid sequence of SEQ ID NO: 7;
(b) an antibody comprising a heavy chain variable region comprising an amino acid sequence of SEQ ID NO: 8 and a light chain variable region comprising an amino acid sequence of SEQ ID NO: 9;
(c) an antibody comprising a heavy chain variable region comprising an amino acid sequence of SEQ ID NO: 10 and a light chain variable region comprising an amino acid sequence of SEQ ID NO: 11;
(d) an antibody comprising a heavy chain variable region comprising an amino acid sequence of SEQ ID NO: 12 and a light chain variable region comprising an amino acid sequence of SEQ ID NO: 13;
(e) an antibody comprising a heavy chain variable region comprising an amino acid sequence of SEQ ID NO: 14 and a light chain variable region comprising an amino acid sequence of SEQ ID NO: 15;
(f) an antibody comprising a heavy chain variable region comprising an amino acid sequence of SEQ ID NO: 16 and a light chain variable region comprising an amino acid sequence of SEQ ID NO: 17; and
(g) an antibody comprising a heavy chain variable region comprising an amino acid sequence of SEQ ID NO: 18 and a light chain variable region comprising an amino acid sequence of SEQ ID NO: 19.

According to any of the previous embodiments,
(**1**) the antibody may specifically bind a human CD39 (NTPDase1) polypeptide comprising an amino acid sequence of SEQ ID NO: 1; and/or
(**2**) the antibody may substantially lack binding, via an Fc domain, to the human Fcγ receptors CD16A, CD16B, CD32A, CD32B and/or CD64; and/or
(**3**) the VH and VL domains may be fused, respectively, to human heavy and light chain constant domains; and/or
(**4**) the antibody may be a full-length antibody comprising an Fc domain; and/or
(**5**) the antibody may comprise a modified human IgG1 Fc domain comprising N-linked glycosylation at Kabat residue N297 and comprising an amino acid substitution at Kabat residue(s) 234 and 235; and/or
(**6**) the antibody may comprise a modified human IgG1 Fc domain comprising N-linked glycosylation at Kabat residue N297 and comprising an amino acid substitution at Kabat residue(s) 234, 235 and 237.

In relation to the antibody according to embodiment (**5**) or (**6**), the Fc domain may further comprise an amino acid substitution at Kabat residue(s) 330 and/or 331 (**7**).

In relation to the antibody according to any of embodiments (**4**) to (**7**), the modified human IgG1 Fc domain may comprise a set of substitutions selected from the group consisting of: L234A/L235E/P331S substitutions, L234F/L235E/P331S substitutions, L234A/L235E/G237A/P331S substitutions, and L234A/L235E/G237A/A330S/P331S substitutions (**8**).

In relation to the antibody according to any of embodiments (**4**) to (**8**), the IgG1 Fc domain may comprise an amino acid sequence of SEQ ID NO: 30, 31, 32 or 33 (**9**).

According to any of the previous embodiments of (II) and (III), the antibody may comprise a heavy chain comprising an amino acid sequence of SEQ ID NOS: 26, 27, 28 or 29, and a light chain comprising an amino acid sequence of SEQ ID NO: 9.

According to any of the previous embodiments,
(**10**) the antibody may be capable of causing a decrease in the ATPase activity of human cellular CD39 polypeptide by more than 70%, optionally more than 80%; and/or
(**11**) the antibody may be characterized by an EC₅₀, as determined by flow cytometry, of no more than 2 µg/ml, optionally no more than 1 µg/ml, no more than 0.5 µg/ml, no more than 0.1 µg/ml, for binding to cells made to express at their surface a CD39 polypeptide comprising an amino acid sequence of SEQ ID NO: 1; and/or
(**12**) the antibody may be capable of causing a decrease in the ATPase activity of a solumble human CD39 polypeptide by more than 50%, optionally more than 70%, optionally more than 80%.

In other aspects, provided are:
a pharmaceutical composition comprising an antibody as defined above, and a pharmaceutically acceptable carrier;
a kit comprising the antibody as defined above, optionally further comprising a labeled secondary antibody that specifically recognizes the antibody as defined above;
a nucleic acid encoding a heavy and/or light chain of an antibody as defined above;
a recombinant host cell producing the antibody as defined above or comprising nucleic acid as defined above.

In other aspects, provided are:
a method for the treatment or prevention of a disease in a patient in need thereof, the method comprising administering to said patient an effective amount of an antibody as defined above or a composition as defined above, advantageously wherein said disease is cancer;
a method for increasing T cell activity in a subject having a cancer, the method comprising administering to said subject an effective amount of an antibody as defined above or a composition as defined above;
a method for relieving adenosine-mediated inhibition of T cell activity in a subject having a cancer, the method comprising administering to said subject an effective amount of an antibody as defined above or a composition as defined above;
a method for increasing T cell activity in the tumor microenvironment of in a subject, the method comprising administering to said subject an effective amount of an antibody as defined above or a composition as defined above;
a method for the treatment or prevention of a cancer in an individual in need thereof, the method comprising:
   a) determining the CD39 polypeptide status of cells within the tumor environment, optionally within the tumor and/or within adjacent tissue, optionally tumor cells, and
   b) upon a determination that tumor environment comprises cells that express CD39 polypeptide, optionally at a level that is increased compared to a reference level, administering to the individual an antibody as defined above or a composition as defined above,
advantageously wherein determining the CD39 polypeptide status within the tumor environment comprises obtaining from the individual a biological sample that comprises cancer tissue and/or adjacent tissue, bringing said cells into contact with an antibody that binds a CD39 polypeptide, and detecting cells that express CD39.

In relation to the method according to any of the embodiments, the anti-CD39 antibody may be administered at least once in an amount effective to achieve a concentration in blood (serum) or a tumor tissue that corresponds to at least the EC₅₀ for neutralization of the enzymatic activity of CD39,
advantageously neutralization of the enzymatic activity of CD39 may be determined by assessing neutralization of ATPase activity in CD39-expressing cells, optionally B cells, optionally Ramos cells, by quantifying the reduction in AMP generated when CD39-expressing cells are incubated with an anti-CD39 antibody.

In relation to the method according to any of the embodiments, the tumor or cancer may be a solid tumor, advantageously the tumor or cancer may be a leukemia, bladder cancer, glioma, glioblastoma, ovarian cancer, melanoma, prostate cancer, thyroid cancer, esophageal cancer or a breast cancer.

## Claims

1. An antibody that binds a human CD39 polypeptide expressed by a cell and that neutralizes the ATPase activity thereof, selected from the group consisting of:
(a) an antibody comprising a heavy chain comprising an amino acid sequence of SEQ ID NO: 26 and a light chain comprising an amino acid sequence of SEQ ID NO: 9;
(b) an antibody comprising a heavy chain comprising an amino acid sequence of SEQ ID NO: 27 and a light chain comprising an amino acid sequence of SEQ ID NO: 9;
(c) an antibody comprising a heavy chain comprising an amino acid sequence of SEQ ID NO: 28 and a light chain comprising an amino acid sequence of SEQ ID NO: 9; and
(d) an antibody comprising a heavy chain comprising an amino acid sequence of SEQ ID NO: 29 and a light chain comprising an amino acid sequence of SEQ ID NO: 9.

2. An antibody that binds human CD39 and that neutralizes the ATPase activity thereof, selected from the group consisting of:
(a) an antibody comprising a heavy chain variable region comprising an amino acid sequence of SEQ ID NO: 6 and a light chain variable region comprising an amino acid sequence of SEQ ID NO: 7;
(b) an antibody comprising a heavy chain variable region comprising an amino acid sequence of SEQ ID NO: 8 and a light chain variable region comprising an amino acid sequence of SEQ ID NO: 9;
(c) an antibody comprising a heavy chain variable region comprising an amino acid sequence of SEQ ID NO: 10 and a light chain variable region comprising an amino acid sequence of SEQ ID NO: 11;
(d) an antibody comprising a heavy chain variable region comprising an amino acid sequence of SEQ ID NO: 12 and a light chain variable region comprising an amino acid sequence of SEQ ID NO: 13;
(e) an antibody comprising a heavy chain variable region comprising an amino acid sequence of SEQ ID NO: 14 and a light chain variable region comprising an amino acid sequence of SEQ ID NO: 15;
(f) an antibody comprising a heavy chain variable region comprising an amino acid sequence of SEQ ID NO: 16 and a light chain variable region comprising an amino acid sequence of SEQ ID NO: 17; and
(g) an antibody comprising a heavy chain variable region comprising an amino acid sequence of SEQ ID NO: 18 and a light chain variable region comprising an amino acid sequence of SEQ ID NO: 19.

3. The antibody of claims 1 or 2, wherein the antibody substantially lacks binding, via an Fc domain, to the human Fcγ receptors CD16A, CD16B, CD32A, CD32B and/or CD64.

4. The antibody of any of the above claims, wherein the VH and VL domains are fused, respectively, to human heavy and light chain constant domains.

5. The antibody of any of the above claims, wherein the antibody is a full-length antibody comprising an Fc domain.

6. The antibody of any of the above claims, wherein the antibody comprises a modified human IgG1 Fc domain comprising N-linked glycosylation at Kabat residue N297 and comprising an amino acid substitution at Kabat residue(s) 234 and 235.

7. The antibody of claim6, wherein the modified human IgG1 Fc domain comprises a set of substitutions selected from the group consisting of: L234A/L235E/P331S substitutions, L234F/L235E/P331S substitutions, L234A/L235E/G237A/P331S substitutions, and L234A/L235E/G237A/A330S/P331S substitutions.

8. The antibody of claim 6 or 7, wherein the IgG1 Fc domain comprises an amino acid sequence of SEQ ID NO: 30, 31, 32 or 33.

9. The antibody of claims 2-8, wherein the antibody comprises a heavy chain comprising an amino acid sequence of SEQ ID NOS: 26, 27, 28 or 29, and a light chain comprising an amino acid sequence of SEQ ID NO: 9.

10. A pharmaceutical composition comprising an antibody according to any one of the above claims, and a pharmaceutically acceptable carrier.

11. A nucleic acid encoding a heavy and/or light chain of an antibody of any one of claims 1 to 9.

12. A recombinant host cell producing the antibody of any one of claims 1 to 9 or comprising a nucleic acid of claim 11.

13. An antibody of any one of claims 1-9 or a composition of claim 10, for use in the treatment or prevention of a cancer in a patient in need thereof.

14. An antibody of any one of claims 1-9 or a composition of claim 10, for use in increasing T cell activity in a subject having a cancer.

15. An antibody of any one of claims 1-9 or a composition of claim 10, for use in the treatment of a cancer in an individual in need thereof, the treatment comprising:
a) determining the CD39 polypeptide status of cells within the tumor environment, optionally within the tumor and/or within adjacent tissue, optionally tumor cells, and
b) upon a determination that tumor environment comprises cells that express CD39 polypeptide, optionally at a level that is increased compared to a reference level, administering to the individual the antibody of any one of claims 1-9 or the composition of claim 10.
